# EUROPEAN PATENT APPLICATION

(11) **EP 3 995 491 A1**
(43) Date of publication of application: **11.05.2022**
(21) Application number: 20836829.0
(22) Date of filing: 19.06.2020
(51) Int. Cl.: C07D 209/80, C08F 20/18, G02B 1/04, G02B 3/00, G03H 1/02

(54) **COMPOUND, POLYMER, ORGANIC MATERIAL, AND OPTICAL DEVICE, OPTICAL COMPONENT, AND IMAGE DISPLAY DEVICE ALL INCLUDING SAID ORGANIC MATERIAL**

(30) Priority: 08.07.2019 JP 2019126973
(71) Applicant: Sony Group Corporation, Tokyo 108-0075 (JP)
(72) Inventor: OHE Takahiro, Tokyo 108-0075 (JP); HARA Hisaya, Tokyo 108-0075 (JP); KAWASAKI Kenshiro, Tokyo 108-0075 (JP)
(74) Representative: D Young & Co LLP
(86) International application number: PCT/JP2020/024149
(87) International publication number: WO 2021/006011

(57) **Abstract**

To provide a compound that is capable of improving the function of an organic material.
The present technology provides a compound represented by the following general formula (1). 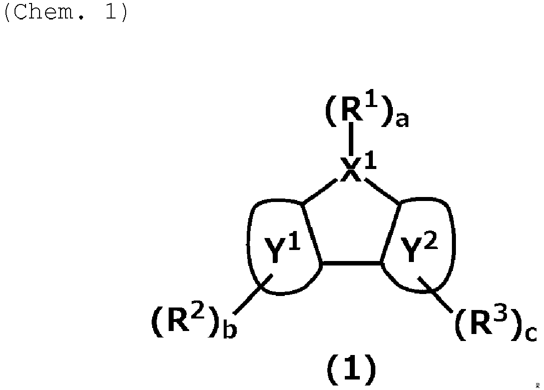 In the general formula (1), X¹ represents an oxygen atom, a nitrogen atom, a phosphorus atom, a caron atom, or a silicon atom.
Y¹ and Y² each represent a benzene ring or a naphthalene ring, and both Y¹ and Y² do not represent benzene rings.
R¹ to R³ each represent a hydrogen or a substituent group represented by *-Z¹(R⁴)_{d} (* represents a bonding site).
Z¹ represents a single bond, a saturated hydrocarbon group having a valence of 2 or higher, or an unsaturated hydrocarbon group having a valence of 2 or higher, the saturated hydrocarbon group or the unsaturated hydrocarbon group optionally having an ether bond and/or a thioether bond.
R⁴ represents a hydrogen or a polymerizable substituent group.

## Description

### Technical Field

The present technology relates to a compound, a polymer, and an organic material, and to an optical apparatus, an optical part, and an image display apparatus using the organic material.

### Background Art

Since a highly functional organic material is excellent in design flexibility and impact resistance and light in weight compared with an inorganic material, application thereof to an optical material such as n organic thin film, an organic lens, and a hologram have been actively studied at present.

For example, in Patent Literature 1, a method of using a compound having a dibenzothiophene skeleton to impart a refractive index of an article has been proposed. Further, in Patent Literature 2, a refractive index improver containing a compound having a dinaphthothiophene skeleton has been proposed.

### Citation List

### Patent Literature

Patent Literature 1: Japanese Patent Application Laid-open No. 2011-162584
Patent Literature 2: Japanese Patent Application Laid-open No. 2011-178985

### Disclosure of Invention

### Technical Problem

However, it is desired to further improve the function of the organic material. In this regard, it is a main object of the present technology to provide a compound and a polymer that are capable of further improving the function of an organic material, and the highly functional organic material, and to provide an optical apparatus, an optical part, and an image display apparatus using the organic material.

### Solution to Problem

The present inventors have intensively studied in order to achieve the above-mentioned object, and thus have succeeded in developing a compound and a polymer that are capable of further improving the function of an organic material, and the highly functional organic material, and developing an optical apparatus, an optical part, and an image display apparatus using the organic material, thereby completing the present technology.

That is, the present technology provides a compound represented by the following general formula (1).

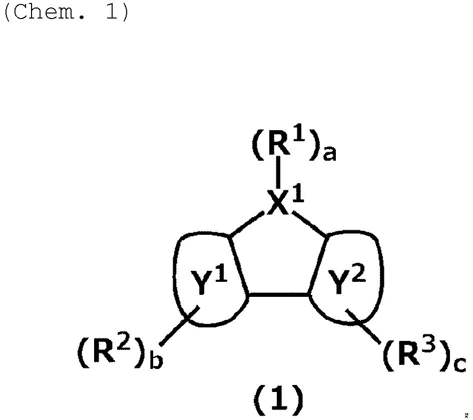

In the general formula (1), X¹ represents an oxygen atom, a nitrogen atom, a phosphorus atom, a caron atom, or a silicon atom. Where X¹ represents an oxygen atom, where X¹ represents a nitrogen atom or a phosphorus atom, and where X¹ represents a caron atom or a silicon atom, a represents 0, a represents 1, and a represents 2, respectively.

Y¹ and Y² each represent a benzene ring or a naphthalene ring, and both Y¹ and Y² do not represent benzene rings. Where Y¹ or Y² represents a benzene ring, b or c corresponding to Y¹ or Y² that is the benzene ring represents 4. Where Y¹ and/or Y² represent(s) a naphthalene ring(s), b and/or c corresponding to Y¹ and/or Y² that is(are) the naphthalene ring(s) represent(s) 6.

R¹ to R³ each represent a hydrogen or a substituent group represented by *-Z¹(R⁴)_{d} (* represents a bonding site). Where R¹ to R³ respectively include a plurality of R¹, a plurality of R², and a plurality of R³, the plurality of R¹ to R³ may be the same or different from each other, but all of R¹ to R³ in the general formula (1) do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other but all R⁴ in the general formula (1) do not represent hydrogens.

X¹ in the general formula (1) may represent a nitrogen atom.

Further, the compound may be a compound represented by the following general formula (1-1).

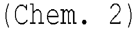

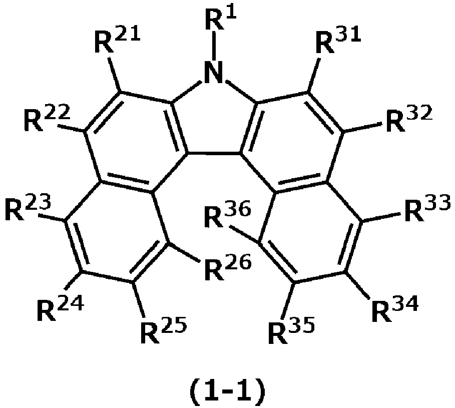

In the general formula (1-1), R¹, R²¹ to R²⁶, and R³¹ to R³⁶ each represent a hydrogen or a substituent group represented by *-Z¹(R⁴)_{d} (* represents a bonding site) . R¹, R²¹ to R²⁶, and R³¹ to R³⁶ may be the same or different from each other. However, all of R¹, R²¹ to R²⁶, and R³¹ to R³⁶ in the general formula (1-1) do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other but all R⁴ in the general formula (1-1) do not represent hydrogens.

However, R¹ represents *-CH=CH₂ and all of R²¹ to R²⁶ and R³¹ to R³⁶ represent hydrogens is not included.

The present technology provides also an organic material containing the compound.

The organic material may be an organic thin film, an organic lens, or a hologram.

Further, the organic material may be a composition for an organic thin film, a composition for an organic lens, or a photosensitive composition for recording a hologram.

The present technology provides also a polymer obtained by polymerizing the compound.

Further, the present technology provides also a co-polymer obtained by the compound and another polymerizable compound.

Further, the present technology provides also an organic material containing the polymer.

The organic material may be an organic thin film, an organic lens, or a hologram.

Further, the organic material may be a composition for an organic thin film, a composition for an organic lens, or a photosensitive composition for recording a hologram.

The present technology provides also an optical apparatus containing the organic material.

Further, the present technology provides also an optical part containing the organic material.

Further, the present technology provides an image display apparatus containing the organic material.

The present technology provides also a composition containing the compound and a radical polymerizable monomer.

The radical polymerizable monomer may be one or more selected from the group consisting of a carbazole monomer, a dinaphthothiophene monomer, a fluorene monomer, and a dibenzofuran monomer.

In accordance with the present technology, it is possible to further improve the function of an organic material.

### Brief Description of Drawings

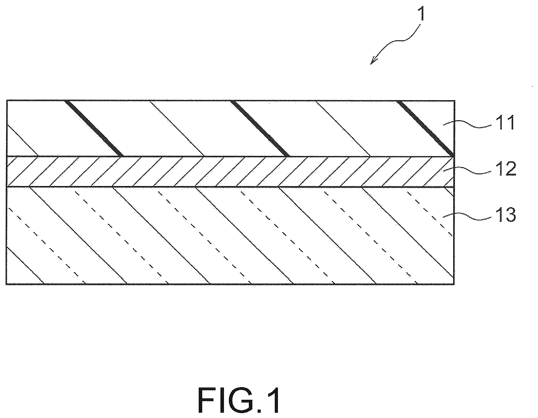

[Fig. 1] Fig. 1 is a cross-sectional view schematically showing an example of a hologram recording medium according to an embodiment of the present technology.

### Mode(s) for Carrying Out the Invention

Hereinafter, suitable embodiments for carrying out the present technology will be described. Note that the embodiments to be described below show representative embodiments of the present technology, and the scope of the present technology is not limited to these embodiments.

Note that the description of the present technology will be given in the following order.
1. Overview of present technology
2. First embodiment (example of compound)
3. Second embodiment (example of polymer)
4. Third embodiment (example of organic material)
   4-1. Organic thin film and composition for organic thin film
   4-2. Organic lens and composition for organic lens
   4-3. Composition for recording hologram, hologram recording medium, and hologram
5. Fourth embodiment (example of optical apparatus) and fifth embodiment (example of optical part)
6. Sixth embodiment (example of image display apparatus)
7. Seventh embodiment (example of composition)

### <1. Overview of present technology>

First, an overview of the present technology will be described.

The present technology relates to a compound, a polymer, and an organic material, and to an optical apparatus, an optical part, and an image display apparatus using the organic material.

An organic compound and a polymer are referred to as high refractive index materials when, for example, the refractive indexes thereof exceed 1.5. However, in the case of applying a compound having a high refractive index to an optical material, the following facts exist.
- It has low solubility in an organic solvent, and it is difficult to perform deposition using a solution.
- The compatibility with a resin is poor, and the compound concentration in a mixture cannot be increased.
- Part of the compound is colored, and it is not suitable for application to a transparent thin film or a lens.

As described above, a compound having a high functionality such as having a high refractive index and being excellent in transparency and solubility in an organic solvent is desired. The present inventors have intensively studied, and thus have found that an organic compound that has a specific skeleton and has a polymerizable substituent group has a high refractive index and is excellent in transparency and solubility in an organic solvent.

### <2. First embodiment (example of compound)>

A compound according to a first embodiment of the present technology is a compound represented by the following general formula (1). The compound according to the first embodiment of the present technology is capable of further improving the function of an organic material. That is, the compound according to the first embodiment of the present technology has a high refractive index and is excellent in transparency and solubility in an organic solvent.

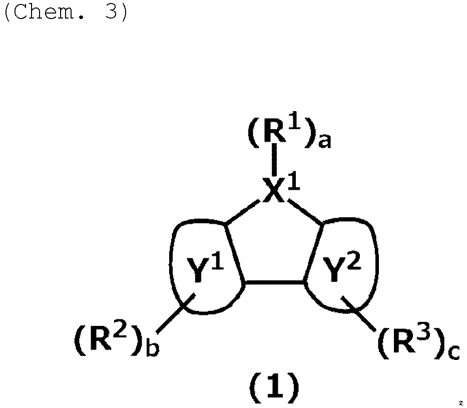

In the general formula (1), X¹ represents an oxygen atom, a nitrogen atom, a phosphorus atom, a caron atom, or a silicon atom. Where X¹ represents an oxygen atom, where X¹ represents a nitrogen atom or a phosphorus atom, and where X¹ represents a caron atom or a silicon atom, a represents 0, a represents 1, and a represents 2, respectively.

Y¹ and Y² each represent a benzene ring or a naphthalene ring, and both Y¹ and Y² do not represent benzene rings. Where Y¹ or Y² represents a benzene ring, b or c corresponding to Y¹ or Y² that is the benzene ring represents 4. Where Y¹ and/or Y² represent(s) a naphthalene ring(s), b and/or c corresponding to Y¹ and/or Y² that is(are) the naphthalene ring(s) represent(s) 6.

R¹ to R³ each represent a hydrogen or a substituent group represented by *-Z¹(R⁴)_{d} (* represents a bonding site). Where R¹ to R³ respectively include a plurality of R¹, a plurality of R², and a plurality of R³, the plurality of R¹ to R³ may be the same or different from each other, but all of R¹ to R³ in the general formula (1) do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other but all R⁴ in the general formula (1) do not represent hydrogens.

In the general formula (1), X¹ represents an oxygen atom, a nitrogen atom, a phosphorus atom, a caron atom, or a silicon atom. Further, it is considerable that the effects of the present technology can be expected also in the group 14 element, the group 15 element, and the group 16 element other than the above (except for the transitional metal).

Among the atoms described above, an oxygen atom, a nitrogen atom, and a caron atom, which are typical elements of an organic compound, are favorable because of the ease of synthesizing a compound, and the respective atom refractive indexes are an oxygen atom: 1.6 to 2.2, a nitrogen atom: 3.5 to 4.4, and a caron atom: 1.7 to 2.4 ("KOGAKU", the Japanese Journal of Optics, Vol. 44 No. 8, 2015, p298-303). In this embodiment, from the viewpoint of obtaining a compound having a high refractive index, X¹ in the general formula (1) is favorably a nitrogen atom having a high value of an atom refractive index.

That is, the compound according to this embodiment may have the following structure.

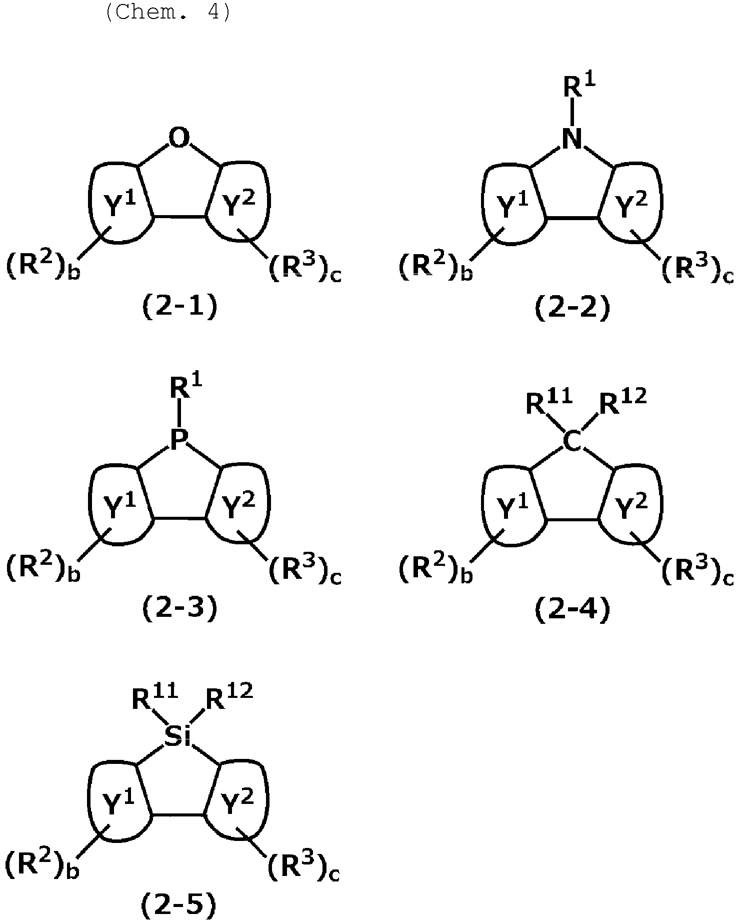

In the general formulae (2-1) to (2-5), Y¹ and Y² each represent a benzene ring or a naphthalene ring, and both Y¹ and Y² do not represent benzene rings. Where Y¹ or Y² represents a benzene ring, b or c corresponding to Y¹ or Y² that is the benzene ring represents 4. Where Y¹ and/or Y² represent(s) a naphthalene ring(s), b and/or c corresponding to Y¹ and/or Y² that is (are) the naphthalene ring(s) represent(s) 6.

R¹, R², R³, R¹¹, and R¹² each represent a hydrogen or a substituent group represented by *-Z¹(R⁴)_{d} (* represents a bonding site). Where R¹ to R³ include a plurality of R¹, a plurality of R², and a plurality of R³, the plurality of R¹ to the plurality of R³ may be the same or different from each other, but all of R¹, R², R³, R¹¹, and R¹² in the general formulae (2-1) to (2-5) do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other, but all of R⁴ in the general formulae (2-1) to (2-5) do not represent hydrogens.

Further, in the general formula (1) described above, Y¹ and Y² each represent a benzene ring or a naphthalene ring, and both Y¹ and Y² do not represent benzene rings.

The molecular refractions of phenyl (C₆H₅) and naphthyl (C₁₀H₇) are phenyl (C₆H₅) : 25.5 and naphthyl (C₁₀H₇): 43.3 ("KOGAKU", the Japanese Journal of Optics, Vol. 44 No. 8, 2015, p298-303). In this embodiment, from the viewpoint of obtaining a compound having a high refractive index, Y¹ and Y² are favorably naphthalene rings having a high value of molecular refraction.

That is, the compound according to this embodiment may have the following structure.

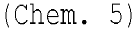

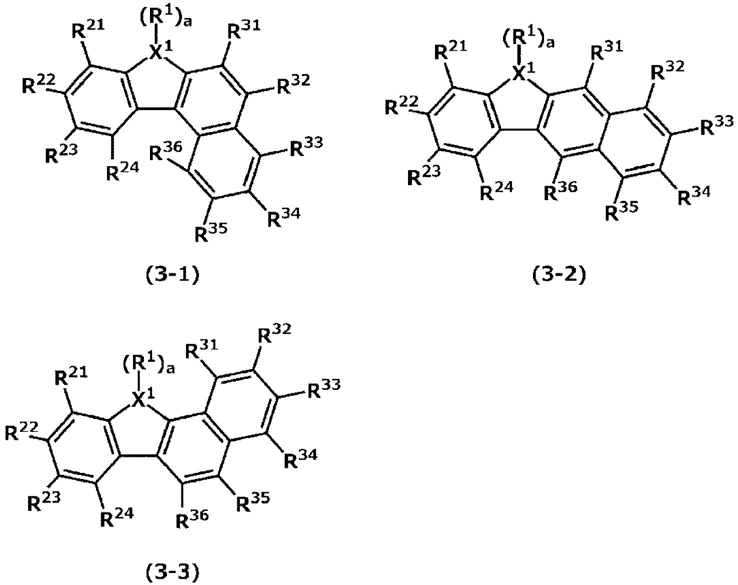

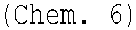

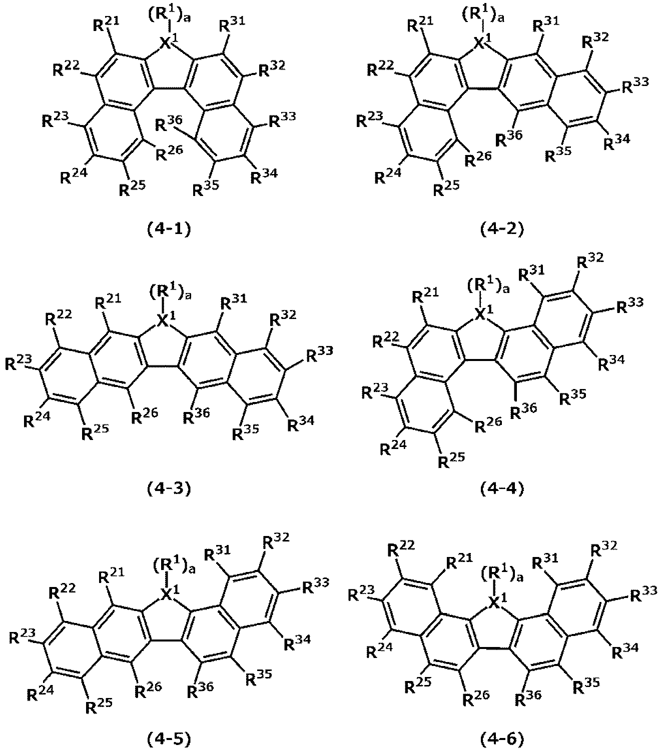

In the general formulae (3-1) to (3-3) and (4-1) to (4-6), X¹ represents an oxygen atom, a nitrogen atom, a phosphorus atom, a caron atom, or a silicon atom. Where X¹ represents an oxygen atom, where X¹ represents a nitrogen atom or a phosphorus atom, and where X¹ represents a caron atom or a silicon atom, a represents 0, a represents 1, and a represents 2, respectively.

R¹, R²¹ to R²⁶, and R³¹ to R³⁶ each represent a hydrogen or a substituent group represented by *-Z¹(R⁴)_{d} (* represents a bonding site) . R¹, R²¹ to R²⁶, and R³¹ to R³⁶ may be the same or different from each other. Further, in the case where R¹ includes a plurality of R¹, the plurality of R¹ may be the same or different from each other. However, all of R¹, R²¹ to R²⁶, and R³¹ to R³⁶ in the general formulae (3-1) to (3-3) and (4-1) to (4-6) do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other, but all of R⁴ in the general formulae (3-1) to (3-3) and (4-1) to (4-6) do not represent hydrogens.

In the general formula (1) described above, Z¹ represents a single bond, a saturated hydrocarbon group having a valence of 2 or higher, or an unsaturated hydrocarbon group having a valence of 2 or higher. The saturated hydrocarbon group or the unsaturated hydrocarbon group may have an ether bond and/or a thioether bond.

Where Z¹ represents a saturated hydrocarbon group having a valence of 2 or higher, the saturated hydrocarbon group may be a linear, branched, or cyclic substituted or unsubstituted hydrocarbon group. In general, an organic compound tends to obtain solubility more easily as the number of simple carbon chains increases, while it tends to have a lower refractive index as the number of simple carbon chains increases. Therefore, the saturated hydrocarbon group favorably has the number of simple carbon chains of 1 to 15, and, more favorably, 1 to 10.

Further, where Z¹ represents an unsaturated hydrocarbon group having a valence of 2 or higher, the unsaturated hydrocarbon group may be a linear, branched, or cyclic substituted or unsubstituted hydrocarbon group, or an aromatic group. The unsaturated hydrocarbon group favorably has the number of simple carbon chains of 1 to 15, and more favorably, 1 to 10. Where the unsaturated hydrocarbon group has an aromatic group, it is favorable that the aromatic group is a substituted or unsubstituted aromatic group having a valence of 2 or higher, which is represented by the following chemical formulae (5-1) to (5-8). When four or more benzene rings are linearly connected, it may be unfavorably from the viewpoint of transparency because it has absorbance in a visible light area and has a color. Therefore, the aromatic group favorably has a structure in which four or more benzene rings are not linearly aligned, and a benzene ring, a naphthalene ring, or an anthracene ring is favorable as a linear shape.

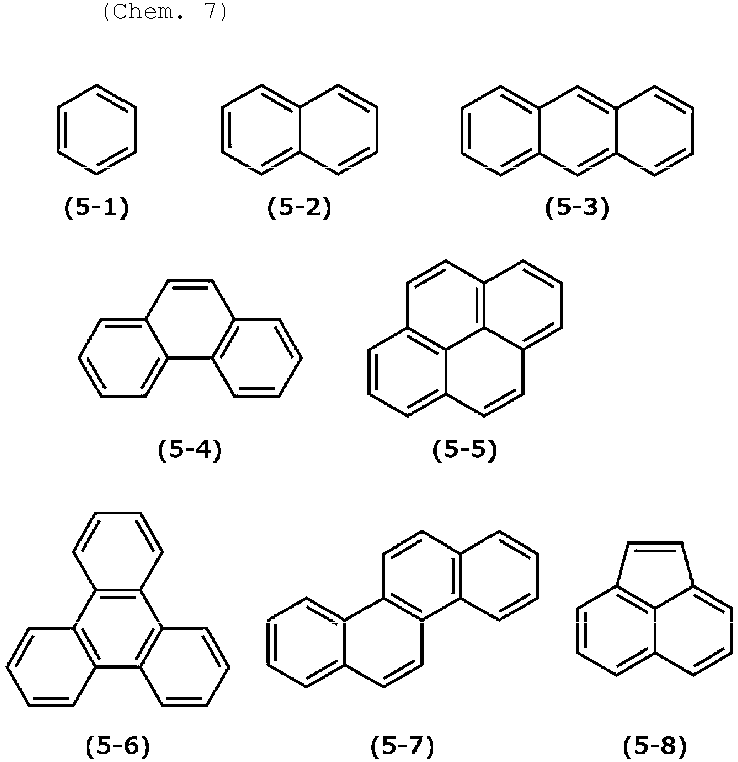

In the general formula (1) described above, examples of the polymerizable substituent group indicated by R⁴ include those having a polymerizable unsaturated group or those having a reactive substituent group. Examples of those having a polymerizable unsaturated group include a vinyl group, an acryl group, a methacrylic group, an acrylamide group, a methacrylamide group, a cyanoacrylate group, a cyanomethacrylate group, a vinyl ether group, a vinyl cyanide group, a nitrated vinyl group, a conjugated polyene group, a halogenated vinyl group, a vinyl ketone group, and a styryl group. Examples of those having a reactive substituent group include an epoxy group, an oxetane group, a hydroxy group, an amino group, a carboxyl group, an acid anhydride group, an acid halide group, and an isocyanate group.

In the general formula (1) described above, it is favorable that X¹ represents a nitrogen atom and Y¹ and Y² each represent a naphthalene ring. That is, the compound is favorably a compound represented by the following general formula (1-1).

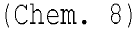

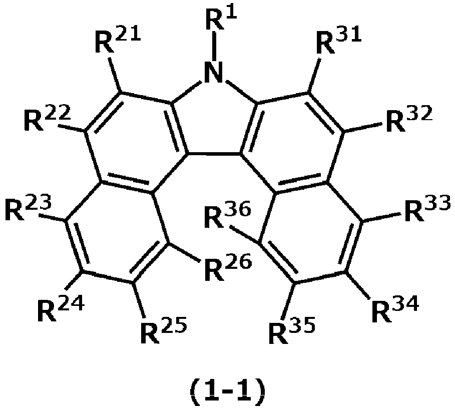

In the general formula (1-1), R¹, R²¹ to R²⁶, and R³¹ to R³⁶ each represent a hydrogen or a substituent group represented by *-Z¹(R⁴)_{d} (* represents a bonding site) . R¹, R²¹ to R²⁶, and R³¹ to R³⁶ may be the same or different from each other. However, all of R¹, R²¹ to R²⁶, and R³¹ to R³⁶ do not represent hydrogens.

Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.

R⁴ represents a hydrogen or a polymerizable substituent group. Where R⁴ includes a plurality of R⁴, the plurality of R⁴ may be the same or different from each other but all R⁴ in the general formula (1-1) do not represent hydrogens.

However, the compound in which R¹ represents *-CH=CH₂ and all of R²¹ to R²⁶ and R³¹ to R³⁶ represent hydrogens is not included.

In the general formula (1-1), it is favorable that R¹ represents a substituent groups represented by *-Z¹(R⁴)_{d} (* represents a bonding site) and R²¹ to R²⁶ and R³¹ to R³⁶ each represent a hydrogen.

In the compound according to this embodiment, the chemical structural formulae of exemplified compounds of favorable monofunctional and polyfunctional (bifunctional) monomers are as follows.

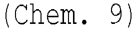

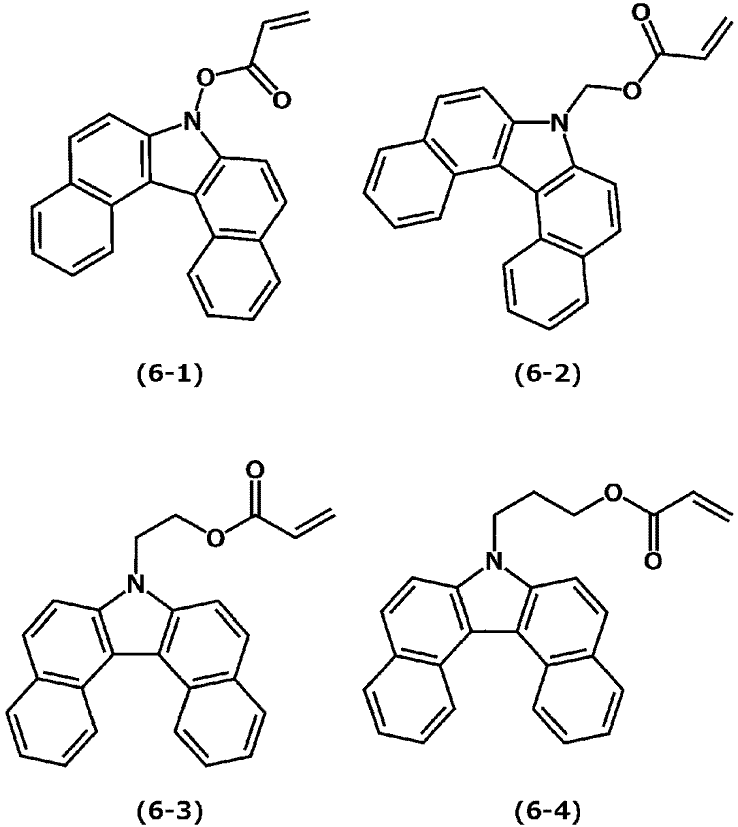

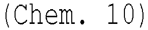

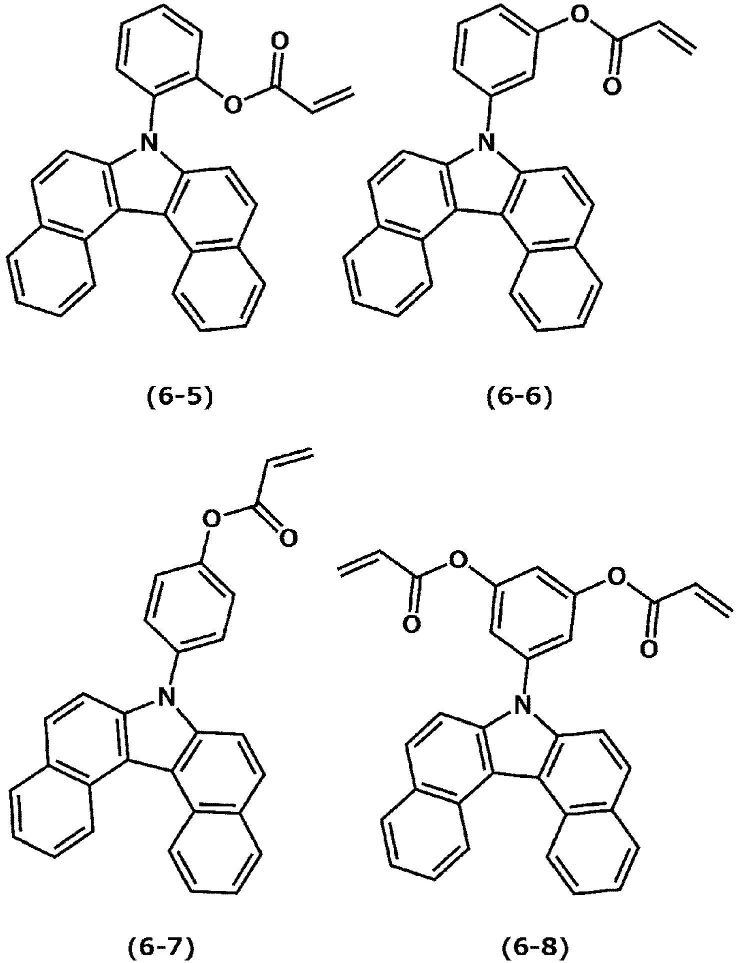

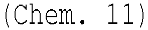

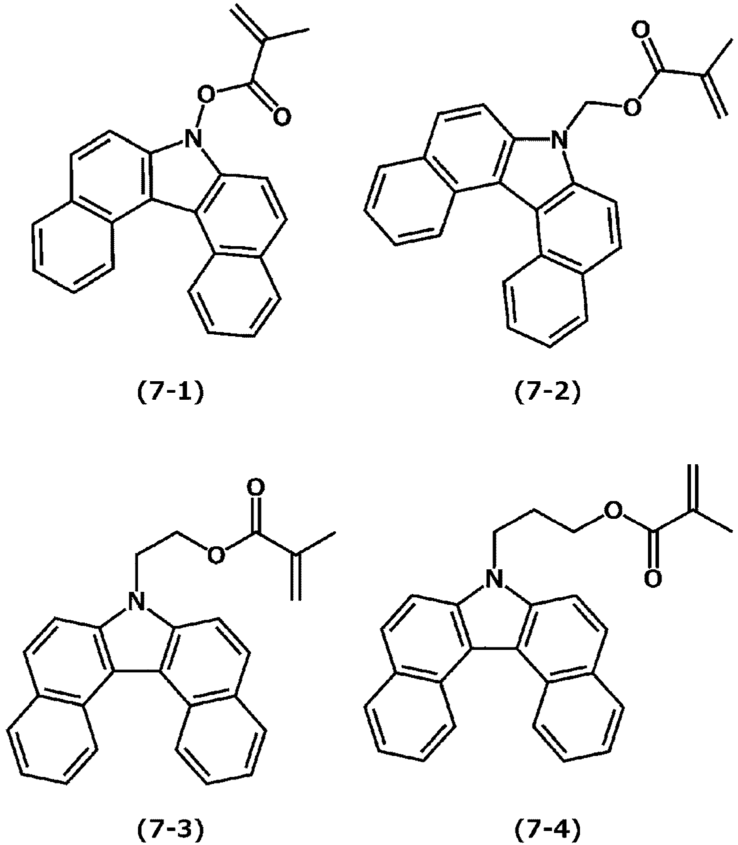

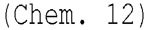

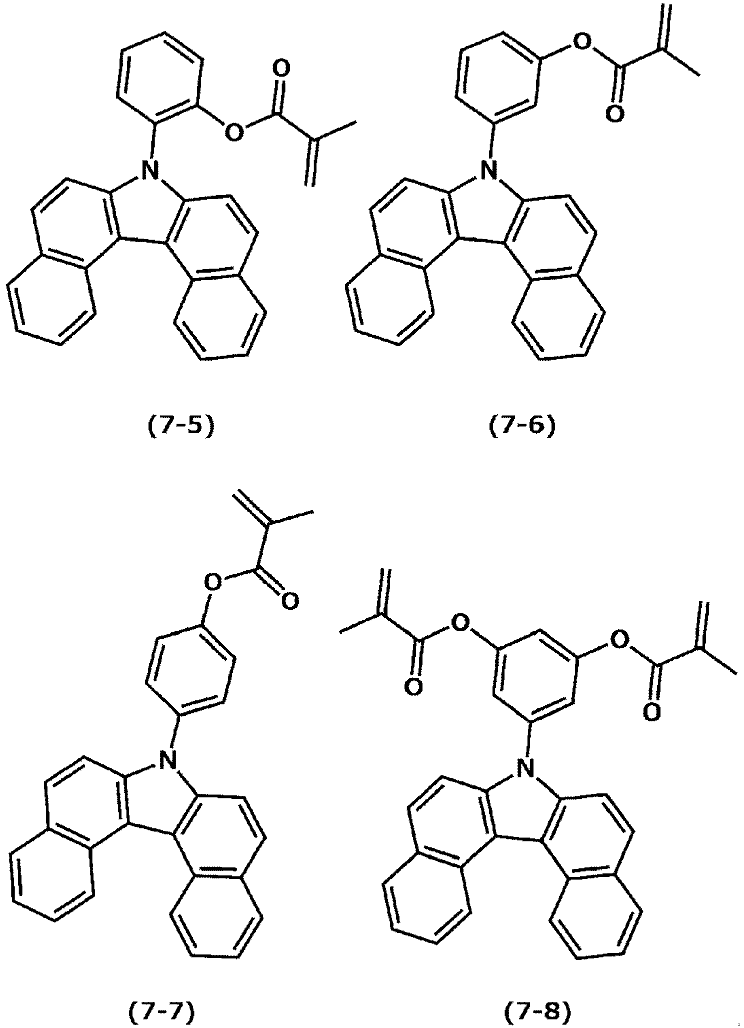

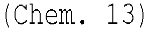

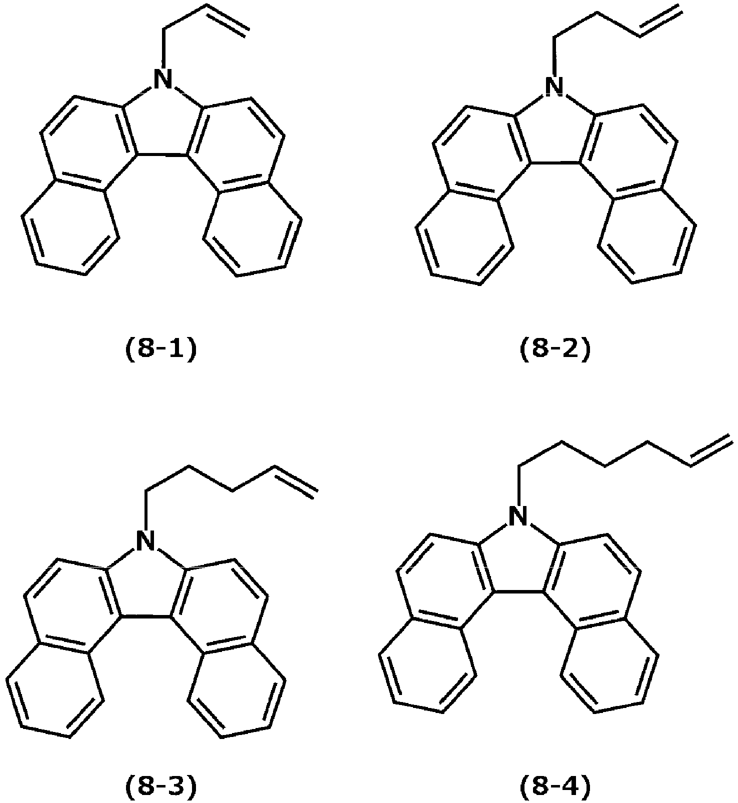

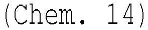

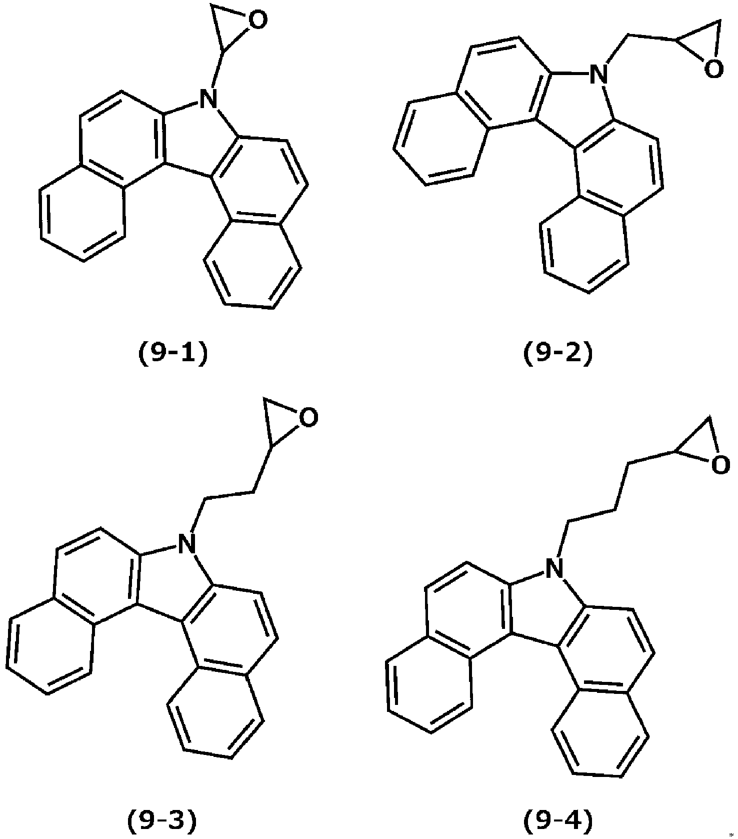

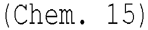

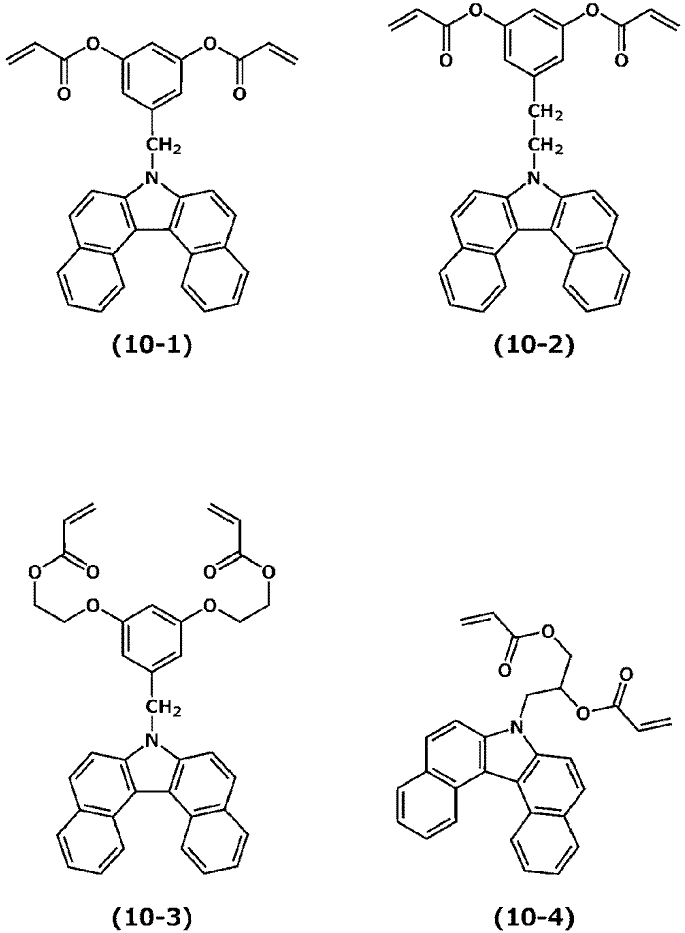

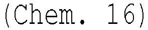

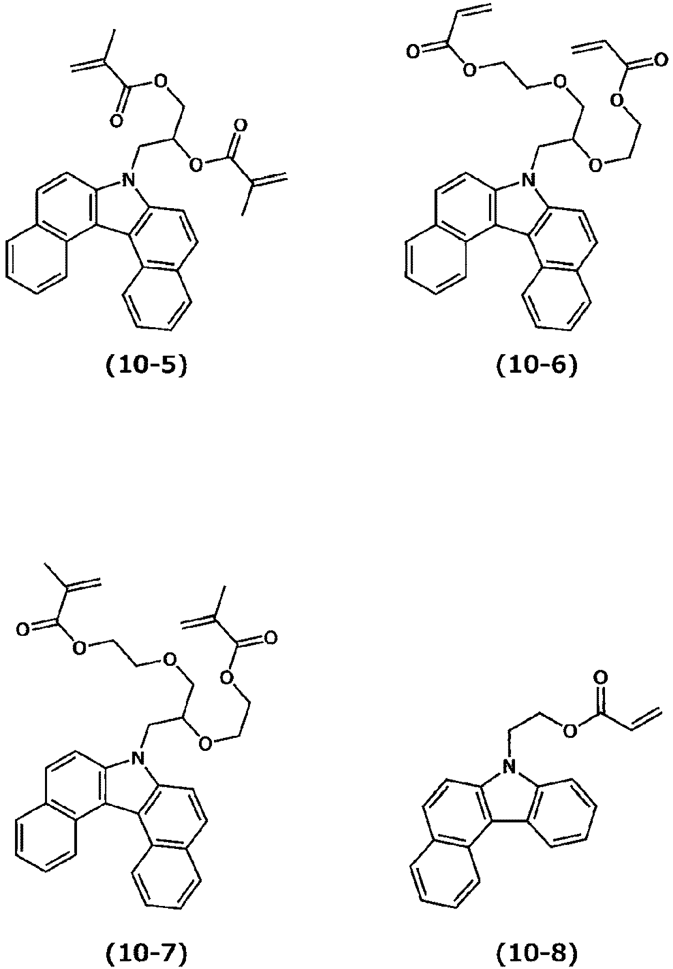

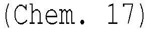

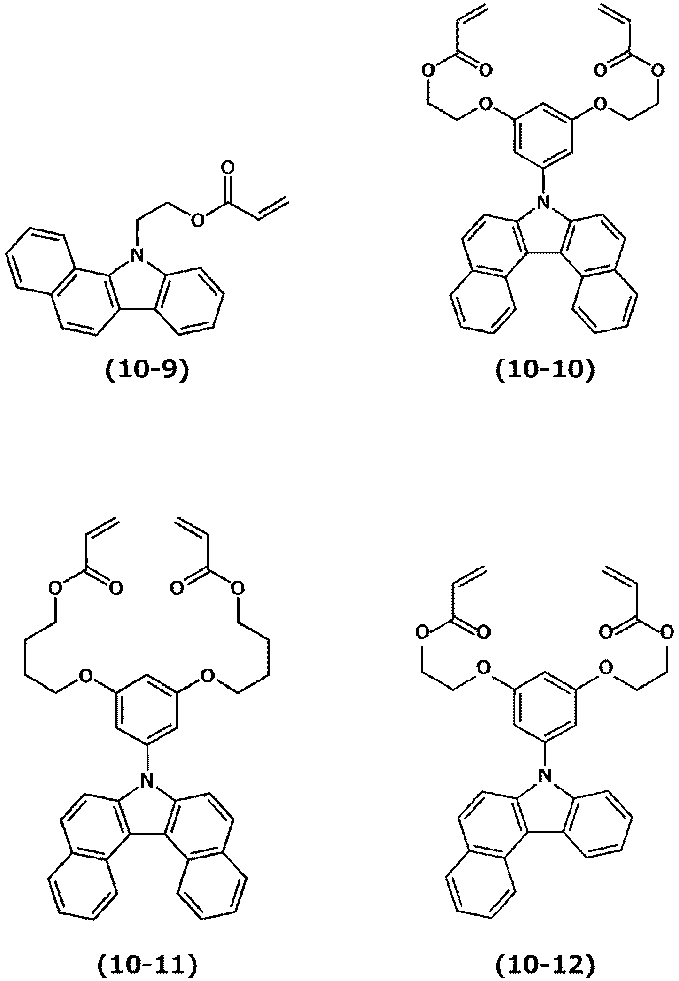

The refractive index of the compound according to this embodiment is favorably 1.60 or more, more favorably 1.65 or more, and still more favorably 1.70 or more. Meanwhile, the refractive index of the compound according to the first embodiment is, for example, 1.80 or less, but may exceed 1.80.

Note that the refractive index can be measured by a critical angle method or a spectroscopic ellipsometry method. For example, in the critical angle method, measurement can be performed using an Abbe refractometer ER-1 manufactured by ERMA INC (measurement wavelengths are measured in visible light areas using 486 nm, 589 nm, 656 nm, and the like).

### <3. Second embodiment (example of polymer)>

A polymer according to a second embodiment of the present technology is a polymer obtained by polymerizing the compound according to the first embodiment of the present technology.

Since the compound according to the first embodiment of the present technology is a monofunctional monomer or a polyfunctional (bifunctional) monomer, the polymer according to the second embodiment of the present technology can be prepared by polymerizing the compound according to the first embodiment of the present technology. Note that in order to adjust the physical properties such as a refractive index, curability, and viscosity of the polymerized product, the compound according to the first embodiment of the present technology and another polymerizable compound may be copolymerized. That is, the polymer according to the second embodiment of the present technology may be a co-polymer obtained by polymerizing the compound according to the first embodiment of the present technology and another polymerizable compound.

The polymer according to the second embodiment of the present technology is capable of further improving the function of an organic material. That is, the polymer according to the second embodiment of the present technology has a high refractive index and is excellent in transparency and solubility in an organic solvent.

### <4. Third embodiment (example of organic material)>

An organic material according to a third embodiment of the present technology is a material containing the compound according to the first embodiment of the present technology or the polymer according to the second embodiment of the present technology.

Examples of the organic material according to the third embodiment of the present technology include an organic thin film, an organic lens, a hologram, a composition for an organic thin film, a composition for an organic lens, and a composition for recording a hologram. An organic thin film and a composition for an organic thin film, an organic lens and a composition for an organic lens, and a hologram and a composition for recording a hologram will be described below in detail.

### [4-1. Organic thin film and composition for organic thin film]

A composition for an organic thin film according to this embodiment contains at least the compound according to the first embodiment of the present technology. The organic thin film can be obtained by subjecting a composition for an organic thin film to polymerization treatment such as photoirradiation and heating. That is, the organic thin film contains the polymer according to the second embodiment of the present technology. The organic thin film is a so-called polymeric film, and a flat-panel display such as a liquid crystal display device (hereinafter, referred to also as an LCD (Liquid Crystal Display)) usually includes one or more layers of the organic thin film.

The organic thin film is incorporated into the flat-panel display as, for example, a protective film in an LCD or a layer constituting an antireflective film. Further, the organic thin film is widely used not only in the flat-panel display but also in various fields that require protective surfaces, preventing reflections, and the like.

Since the compound according to the first embodiment of the present technology has a high refractive index and is excellent in transparency and solubility in an organic solvent, it can be used for an organic thin film (e.g., a refractive index gradient film) having a high-refractive index surface. In order to obtain an organic thin film (e.g., a refractive index gradient film) having a high-refractive index surface, it is favorable that a polymer of the compound according to the first embodiment, which has a refractive index of 1.60 or more, is localized on a surface layer portion on one surface side (high-refractive index surface side) of an organic thin film (polymer film). Further, as the compound according to the first embodiment, two or more of them different from each other can be mixed and used in an arbitrary ratio.

### [4-2. Organic lens and composition for organic lens]

A composition for an organic lens according to this embodiment contains at least a compound according to the first embodiment of the present technology. The organic lens is can be obtained by subjecting a composition for an organic lens to polymerization treatment such as photoirradiation and heating. That is, the organic lens contains the polymer according to the second embodiment of the present technology.

The organic lens has the advantages of being lighter, less susceptible to cracking, and easier to process than an inorganic material, and the organic lens is used for a spectacle and a camera. Since the compound according to the first embodiment of the present technology has a high refractive index and favorable transparency, the compound has an advantage of being excellent in convenience in optical applications when it is used as an organic lens, e.g., the thickness of the lens can be made thinner than that of glass.

### [4-3. Composition for recording hologram, hologram recording medium, and hologram]

### (4-3-1. Composition for recording hologram)

The composition for recording a hologram according to this embodiment is a composition containing at least a photopolymerizable monomer, a photopolymerization initiator, a binder resin, and a polymerization inhibitor. As the photopolymerizable monomer, the compound according to the first embodiment of the present technology can be used. Further, as the photopolymerizable monomer, a radical polymerizable monomer other than the compound according to the first embodiment of the present technology may be contained.

The composition for recording a hologram according to this embodiment has a high functionality, e.g., has a high refractive index modulation amount (Δn), and exhibits the effect of excellent diffraction properties.

In the case where a monomer other than the compound according to the first embodiment of the present technology is used as the photopolymerizable monomer according to this embodiment, an arbitrary photopolymerizable monomer can be used. Examples thereof include a monofunctional or polyfunctional carbazole monomer, a dinaphthothiophene monomer, a fluorene monomer, and a dibenzofuran monomer, and one or two or more of them can be used.

The photopolymerization initiator contained in the composition for recording a hologram according to this embodiment is not particularly limited, and an arbitrary photopolymerization initiator can be used. Favorable examples thereof include one or more radical polymerization initiators (radical generators), cationic polymerization initiators (acid generators), or those having both the functions selected from the group consisting of imidazole-based, bisimidazole-based, N-arylglycine-based, organic azide compound-based, organoboron compound-based, titanocene-based, aluminate complex-based, organic peroxide-based, N-alkoxypyridinium salt-based, thioxanthone derivative-based, sulfonic acid ester-based, imide sulfonate-based, dialkyl-4-hydroxysulfonium salt-based, aryl sulfonic acid-p-nitrobenzyl ester-based, silanol-aluminum complex-based, (η6-benzene) (η5-cyclopentadienyl) iron (II)-based, ketone-based, diaryliodonium salt-based, diaryliodonium organoboron complex-based, aromatic sulfonium salt-based, aromatic diazonium salt-based, aromatic phosphonium salt-based, triazine compound-based, and iron arene complex-based ones. Further, as the photopolymerization initiator, an anionic polymerization initiator (base generator) may be used.

The binder resin contained in the composition for recording a hologram according to this embodiment is not particularly limited, and an arbitrary binder resin can be used. However, a vinyl acetate resin is favorable, and polyvinyl acetate or a hydrolysate thereof is suitably used. Further, an acrylic resin is favorable, and a poly (meth) acrylate ester or a partial hydrolysate thereof is suitably used.

The polymerization inhibitor contained in the composition for recording a hologram according to this embodiment is not particularly limited, and an arbitrary polymerization inhibitor can be used. Examples thereof include quinone-based compound such as hydroquinone; a hindered phenol-based compound; a benzotriazole compound; and a thiazine-based compound such as phenothiazine, and one or two or more of them can be used.

The composition for recording a hologram according to this embodiment may further contain inorganic fine particles, a plasticizer, a sensitizing dye, a chain transfer agent, a solvent, and the like.

Note that the sensitizing dye may contain either or both of a dye having absorption in a visible light area and a UV-sensitizing dye (anthracene compound or the like) to be added for the purpose of improving the photoefficiency at the time of UV irradiation. Further, only 1 kind of a sensitizing dye may be used, and a plurality of kinds of sensitizing dyes may be used to correspond to a plurality of wavelengths.

### (4-3-2. Method of producing composition for recording hologram)

The composition for recording a hologram according to this embodiment can be produced, for example, by adding a photopolymerizable monomer, a photopolymerization initiator, a binder resin, and a polymerization inhibitor in a predetermined amount to the above-mentioned solvent at ordinary temperature or the like, and dissolving and mixing the mixture. Further, in accordance with the application, purpose, and the like, the above-mentioned inorganic fine particles, plasticizer, sensitizing dye, chain transfer agent, and the like may be added. In the case where the composition for recording a hologram according to this embodiment is formed on a transparent base material contained in a hologram recording medium described below, the composition for recording a hologram may be used as coating liquid.

### (4-3-3. Hologram recording medium)

The hologram recording medium according to this embodiment is a hologram recording medium that includes a photocurable resin layer containing the above-mentioned composition for recording a hologram, and at least one transparent base material, a photocurable resin layer being formed on the at least one transparent base material. The hologram recording medium according to this embodiment may have a three-layer structure in which a photocurable resin layer is formed on a first transparent base material and a second transparent base material is formed on the main surface of the photocurable resin layer on which the first transparent base material is not formed.

Here, a schematic cross-sectional view of an example of the hologram recording medium according to this embodiment is shown in Fig. 1. As shown in Fig. 1, a hologram recording medium 1 is configured to have a three-layer structure in which a photocurable resin layer 12 is formed between a transparent protective film (referred to as the first transparent base material in some cases) 11 and a glass or film substrate (referred to as the second transparent base material in some cases) 13.

The hologram recording medium according to this embodiment has a high functionality, e.g., has a high refractive index modulation amount (Δn), and exhibits the effect of excellent diffraction properties.

### (4-3-4.Method of producing hologram recording medium)

The hologram recording medium according to this embodiment can be obtained, for example, by applying coating liquid formed of the above-mentioned composition for recording a hologram onto the transparent base material by using a spin coater, a gravure coater, a comma coater, a bar coater or the like, and then drying them to form a photocurable resin layer.

### (4-3-5. Hologram)

The hologram according to this embodiment has a high functionality, e.g., has a high refractive index modulation amount (Δn), and exhibits the effect of excellent diffraction properties.

### (4-3-6. Method of producing hologram)

The hologram according to this embodiment can be obtained by performing two-beam exposure on the above-mentioned hologram recording medium by using a semiconductor laser in a visible light area and then irradiating the entire surface with UV (ultraviolet rays) to cure the uncured monomers or the like, and fixing the refractive index distribution to the hologram recording medium. The conditions of the two-beam exposure may be appropriately set by those skilled in the art in accordance with the application, the purpose, and the like of the hologram. However, it is desirable to perform interferometric exposure by setting the light intensity of one beam on the hologram recording medium to 0.1 to 100 mW/cm² for 1 to 1000 seconds so that the angle between the two beams is 0.1 to 179.9 degrees.

### <5. Fourth embodiment (example of optical apparatus) and fifth embodiment (example of optical part)>

An optical apparatus according to a fourth embodiment of the present technology is an apparatus containing the organic material according to the third embodiment of the present technology. Since the optical apparatus according to this embodiment contains the organic material according to the third embodiment of the present technology, the optical apparatus exhibits the effects of excellent optical properties and excellent optical stability.

Further, an optical part according to the fifth embodiment of the present technology is a part containing the organic material according to the third embodiment of the present technology. Since the optical part according to this embodiment contains the organic material according to the third embodiment of the present technology, the optical part exhibits the effects of excellent optical properties and excellent optical stability.

Examples of the optical apparatus according to a fourth embodiment of the present technology and optical part according to the fifth embodiment of the present technology include an imaging apparatus, an image sensor, a color filter, a diffraction lens, a light guide plate, a spectroscopic element, a hologram sheet, an information recording medium such as an optical disc and a magneto-optical disc, an optical pick-up device, a polarization microscope, and a sensor.

### <6. Sixth embodiment (example of image display apparatus)>

An image display apparatus according to a sixth embodiment of the present technology is an apparatus containing the organic material according to the third embodiment of the present technology. Since the image display apparatus according to this embodiment contains the organic material according to the third embodiment of the present technology, the image display apparatus exhibits the effect of excellent image display performance.

Examples of the image display apparatus according to this embodiment include an image display apparatus such as an eyewear, a holographic screen, a transparent display, a head-mounted display, and a head-up display.

### <7. Seventh embodiment (example of composition)>

A composition according to a seventh embodiment of the present technology is a composition containing the compound according to the first embodiment of the present technology and a radical polymerizable monomer.

Since the composition according to this embodiment has a high refractive index, the composition can be used for, for example, an organic thin film, an organic lens, an optical film, or a hologram as a high refractive index curable resin.

Note that as the radical polymerizable monomer, an arbitrary radical polymerizable monomer can be used. Examples thereof include a monofunctional or polyfunctional carbazole monomer, a dinaphthothiophene monomer, a fluorene monomer, and a dibenzofuran monomer, and one or two more of them can be used.

### (Example)

The effects of the present technology will be specifically described with reference to Examples. Note that the scope of the present technology is not limited to the Examples.

### <Test Example 1>

### [Preparation of compound represented by chemical formula (6-3)]

A compound represented by the following chemical formula (6-3) was synthesized, and the compound represented by the following chemical formula (6-3) was used as a compound of Test Example 1.

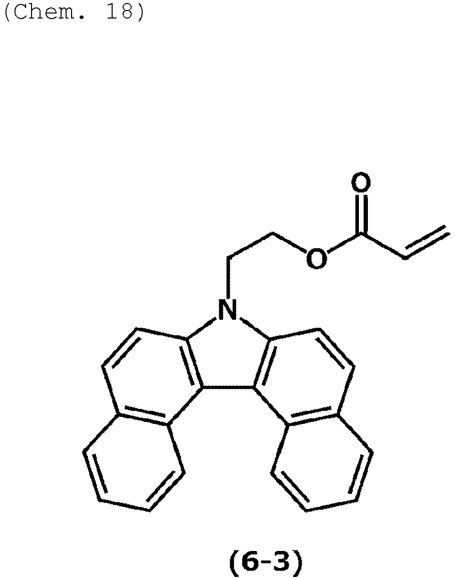

### [Method of synthesizing compound represented by chemical formula (6-3)]

A method of synthesizing (synthesis route of) the compound represented by the chemical formula (6-3) is as follows.

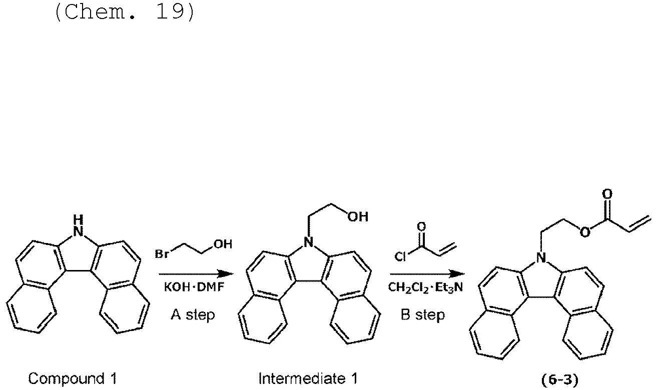

### (A step)

An A step of the synthesis method (synthesis route) described above will be described.

110 mL of a solution of N,N-dimethylformamide (manufactured by Kanto Chemical Co., Ltd.) mixed with 20 g of potassium hydroxide (manufactured by Kanto Chemical Co., Ltd.) was prepared in an inert atmosphere, 15 g of a compound 1 (7H-dibenzo [c, g] carbazole (manufactured by Tokyo Chemical Industry Co., Ltd.)) was added thereto, the mixture was stirred for 1 hours, and then 25 g of 2-bromoethanol (manufactured by Tokyo Chemical Industry Co., Ltd.) was added thereto and reacted for 20 hours. Water was added thereto for quenching, extraction was performed on the mixture with toluene using a separatory funnel, and column purification was performed to obtain 10 g of an intermediate 1 as a target product.

### (B step)

A B step of the synthesis method (synthesis route) described above will be described.

9 g of the intermediate 1 was dissolved in a solution obtained by mixing triethylamine (manufactured by Kanto Chemical Co., Ltd.) in 50 mL of methylene chloride (manufactured by Kanto Chemical Co., Ltd.), and the mixture was cooled in an ice bath. After that, 3 mL of acrylic chloride (manufactured by Tokyo Chemical Industry Co., Ltd.) was added in portions, and the mixture was brought to room temperature at a natural temperature rise and reacted for 4 hours. Water was added thereto for quenching, and extraction was performed on the mixture with methylene chloride (manufactured by Kanto Chemical Co., Ltd.) using a separatory funnel. After that, the organic layer was cleaned with saline, silica filtration was performed, and then, column purification was performed to obtain 6 g of the compound represented by the chemical formula (6-3) .

NMR was used to identify the structure of the compound according to Test Example 1 (the compound represented by the chemical formula (6-3)). The NMR results are as follows.

1H NMR (CDCl₃) : 4.60-4.64 (2H), 4.85-4.89 (2H), 5.74-5.76 (1H), 5.95-6.05 (1H), 6.25-6.31 (1H), 7.49-7.55 (2H), 7.65-7.69 (2H), 7.70-7.77 (2H), 7.91-7.94 (2H), 8.03-8.06 (2H), 9.18-9.22 (2H).

### <Test Example 2>

### [Preparation of compound represented by chemical formula (6-8)]

A compound represented by the following chemical formula (6-8) was synthesized, and the compound represented by the following chemical formula (6-8) was used as a compound according to Test Example 2.

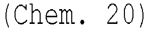

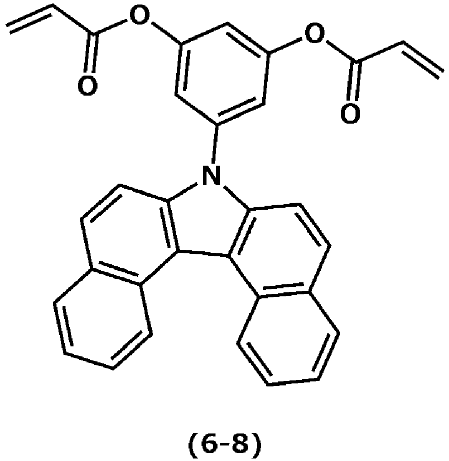

### [Method of synthesizing compound represented by chemical formula (6-8)]

The method of synthesizing (synthesis route of) the compound represented by the chemical formula (6-8) is as follows.

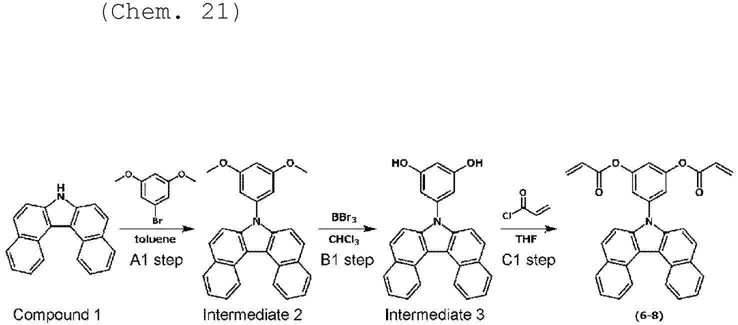

### (A1 step)

An A1 step of the synthesis method (synthesis route) described above will be described.

300 mL of a toluene (manufactured by Kanto Chemical Co., Ltd.) solution of 24 g of 1-bromo-3,5-dimethoxybenzene (manufactured by Tokyo Chemical Industry Co., Ltd.), 36 g of tripotassium phosphate (manufactured by Kanto Chemical Co., Ltd.), and 15 g of the compound 1 (7H-dibenzo [c, g] carbazole (manufactured by Tokyo Chemical Industry Co., Ltd.)) was prepared in an inert atmosphere, 25 mL of 1,2-cyclohexanediamine (manufactured by Tokyo Chemical Industry Co., Ltd.) and 20 g of copper iodide (manufactured by Fujifilm Wako Pure Chemical Industries, Ltd.) were added thereto, and the mixture was reacted at heating reflux to obtain 10 g of an intermediate 2.

### (B1 step)

A B1 step of the synthesis method (synthesis route) described above will be described.

9 g of the intermediate 2 was dissolved in 125 mL of chloroform (manufactured by Kanto Chemical Co., Ltd.), the mixture was cooled with ice water, and then, 100 mL of a dichloromethane solution (concentration of 1 mol/L) in which boron tribromide was dissolved was added dropwise, stirred under ice cooling, and then reacted at room temperature for 1 hour. Ice water was added thereto for quenching, heptane (manufactured by Kanto Chemical Co., Ltd.) was added thereto, and the mixture was recrystallized in a refrigerator to obtain 8 g of an intermediate 3.

### (C1 step)

A C1 step of the synthesis method (synthesis route) described above will be described.

5 g of the intermediate 3 was dissolved in a solution obtained mixing 50 mL of tetrahydrofuran (manufactured by Kanto Chemical Co., Ltd.), 8.5 mL of triethylamine (manufactured by Kanto Chemical Co., Ltd.), and 30 mg of butylhydroxytoluene (manufactured by Tokyo Chemical Industry Co., Ltd.). After that, 3.5 mL of acrylic chloride (manufactured by Tokyo Chemical Industry Co., Ltd.) was added in portions, the mixture was reacted at room temperature for 30 minutes, and then water was added thereto for quenching to obtain 3.5 g of the compound represented by the chemical formula (6-8) by filtration treatment and column purification.

NMR was used to identify the structure of the compound according to Test Example 2 (the compound represented by the chemical formula (6-8)). The NMR results are as follows.

1H NMR (CDCl₃) : 6.05-6.08 (2H), 6.29-6.39 (2H), 6.63-6.69 (2H), 7.25-7.27 (1H), 7.35-7.36 (2H), 7.53-7.54 (2H), 7.69-7.72 (4H), 7.86-7.90 (2H), 8.03-8.06 (2H), 9.21-9.24 (2H).

### <Test Example 3>

### [Preparation of compound represented by chemical formula (10-1)]

A compound represented by the following chemical formula (10-1) was synthesized, and the compound represented by the following chemical formula (10-1) was used as a compound according to Test Example 3.

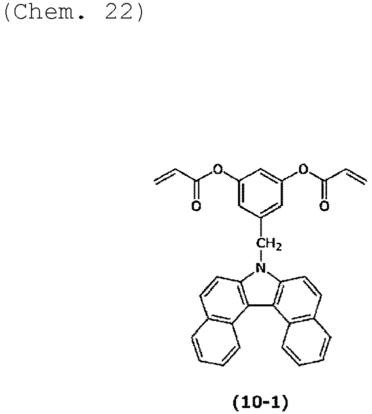

### [Method of synthesizing compound represented by chemical formula (10-1)]

The method of synthesizing (synthesis route of) the compound represented by the chemical formula (10-1) is as follows.

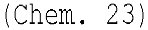

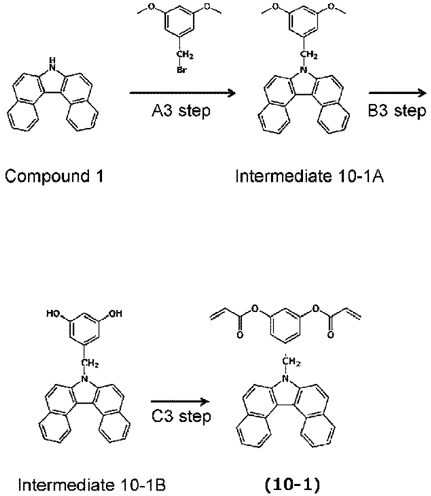

### (A3 step)

In an A3 step of the method of synthesizing (synthesis route of) the compound represented by the chemical formula (10-1), an intermediate 10-1A was obtained by using a method similar to the method shown in the A1 step of the method of synthesizing (synthesis route of) the compound represented by the chemical formula (6-8) except that 1-bromo-3,5-dimethoxybenzene (manufactured by Tokyo Chemical Industry Co., Ltd.) in the A1 step of the method of synthesizing (synthesis route of) the compound represented by the chemical formula (6-8) was replaced with 3,5-dimethoxybenzyl bromide.

### (B3 step)

In a B3 step of the method of synthesizing (synthesis route of) the compound represented by the chemical formula (10-1), an intermediate 10-1B was obtained by using a method similar to the method shown in the B1 step of the method of synthesizing (synthesis route of) the compound represented by the chemical formula (6-8).

### (C3 step)

In a C3 step of the method of synthesizing (synthesis route of) the compound represented by the chemical formula (10-1), the compound represented by the chemical formula (10-1) was obtained by using a method similar to the method shown in the C1 step of the method of synthesizing (synthesis route of) the compound represented by the chemical formula (6-8).

NMR was used to identify the structure of the compound according to Test Example 3(the compound represented by the chemical formula (10-1). The NMR results are as follows.

### (NMR results)

1H NMR (CDCl₃): 5.78-5.79 (2H), 5.92-5.97 (2H), 6.14-6.23 (2H), 6.48-6.54 (2H), 6.77-6.78 (2H), 6.95-6.96 (1H), 7.51-7.56 (2H), 7.63-7.73 (4H), 7.88-7.92 (2H), 8.03-8.06 (2H), 9.22-9.26 (2H).

### <Test Example 4>

### [Preparation of compound represented by chemical formula (10-2)]

A compound represented by the following chemical formula (10-2) was synthesized, and the compound represented by the following chemical formula (10-2) was used as a compound according to Test Example 4.

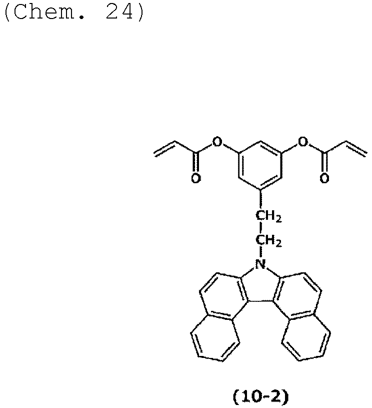

### [Method of synthesizing compound represented by chemical formula (10-2)]

A method of synthesizing (synthesis route of) the compound represented by the chemical formula (10-2) is as follows.

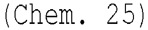

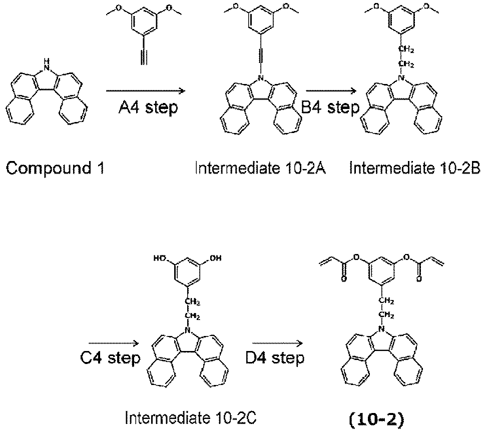

### (A4 step)

An A4 step of the synthesis method (synthesis route) described above will be described.

20 g of the compound 1 (7H-dibenzo [c, g] carbazole (manufactured by Tokyo Chemical Industry Co., Ltd.)), 36.3 g of 1-Ethynyl 3,5-dimethoxybenzene, 300 mL of dehydrated DMSO, and 4.20 g of potassium hydroxide (74.9mmol, 1.00eq) were charged in an inert gas atmosphere, and the mixture was stirred while being heated for several hours. After that, extraction with a separatory funnel and column purification were performed, and then the obtained product was concentrated to dryness under reduced pressure to obtain 17.6 g of an intermediate 10-2A.

### (B4 step)

A B4 step of the synthesis method (synthesis route) described above will be described.

The B4 step of the method of synthesizing (synthesis route of) the compound represented by the chemical formula (10-2) will be described.

17.6 g of the 2L intermediate 10-2A was dissolved in 900 mL of THF in an inert gas atmosphere, 4.82 g of 10% Pd/C(55% water content) (manufactured by Tokyo Chemical Industry Co., Ltd.) was added thereto, and the mixture was reacted with hydrogen gas, follows by filtration and concentration under reduced pressure to obtain 16 g of an intermediate 10-2B as white crystal.

### (C4 step)

In a C4 step of the method of synthesizing (synthesis route of) the compound represented by the chemical formula (10-2), 5 g of an intermediate 10-2C was obtained by using a method similar to the method shown in the B1 step of the method of synthesizing (synthesis route of) the compound represented by the chemical formula (6-8) except that the intermediate 2 in the B1 step of the method of synthesizing (synthesis route of) the compound represented by the chemical formula (6-8) was replaced with the intermediate 10-2B.

### (D4 step)

A D4 step of the synthesis method (synthesis route) described above will be described.

5 g of the intermediate 10-2C, 21 mL of dehydrated toluene (manufactured by Fujifilm Wako Pure Chemical Industries, Ltd.), 291 mg of DMAP, and 1.9 mL of acrylic acid were mixed in an inert gas atmosphere, 4.15 mL of diisopropylcarbodiimide (manufactured by Tokyo Chemical Industry Co., Ltd.) was added thereto while being cooled in an ice bath, the mixture was stirred, and then, 41.5 mL of dehydrated chloroform (manufactured by Fujifilm Wako Pure Chemical Industries, Ltd.) was added to the reaction solution. After the mixture was stirred at room temperature and then separated by chloroform, the mixture was concentrated to dryness under reduced pressure and column purification was performed to obtain 2 g of the compound represented by the chemical formula (10-2).

NMR was used to identify the structure of the compound according to Test Example 4 (the compound represented by the chemical formula (10-2)). The NMR results are as follows.

### (NMR results)

1H NMR (CDCl₃): 3.18-3.23 (2H), 4.76-4.82 (2H), 5.97-6.01 (2H), 6.19-6.29 (2H), 6.50-6.57 (2H), 6.83-6.89 (2H), 6.89-6.90 (1H), 7.48-7.54 (2H), 7.61-7.70 (4H), 7.87-7.91 (2H), 8.03-8.06 (2H), 9.18-9.21 (2H).

### <Test Example 5>

### [Preparation of compound represented by chemical formula (10-3)]

The compound represented by the following chemical formula (10-3) was synthesized, and the compound represented by the following chemical formula (10-3) was used as a compound according to Test Example 5.

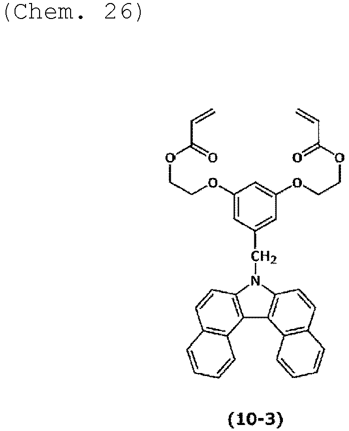

### [Method of synthesizing compound represented by chemical formula (10-3)]

The method of synthesizing (synthesis route of) the compound represented by the chemical formula (10-3) is as follows.

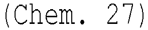

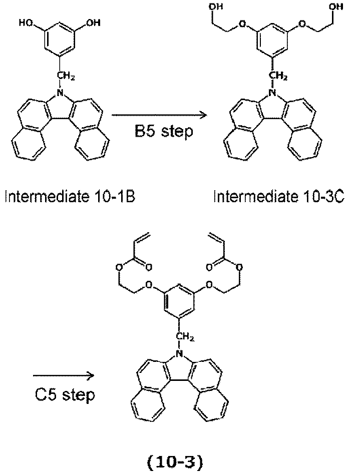

First, synthesis was advanced by using the A3 step and the B3 step of the method of synthesizing (synthesis route of) the compound represented by the chemical formula (10-1) to obtain the intermediate 10-1B.

### (B5 step)

A B5 step of the synthesis method (synthesis route) described above will be described.

The B5 step of the method of synthesizing (synthesis route of) the compound represented by the chemical formula (10-3) will be described. 360 mL of an aqueous sodium hydroxide solution (concentration of 8.0 mol/L, manufactured by Fujifilm Wako Pure Chemical Industries, Ltd.), 13 g of tetrabutylammonium bromide (manufactured by Tokyo Chemical Industry Co., Ltd.), and 40 g of the intermediate 10-1B were mixed in an inert atmosphere, the mixture was cooled with ice water, and then, 70 mL of 2-(2-chloroethoxy)tetrahydro-2H-pyran (manufactured by Tokyo Chemical Industry Co., Ltd.) was added dropwise. The ice water was removed, and the mixture was stirred for 2 hours while being heated to 80°C. Water was used for quenching, and extraction was performed using chloroform. 140 g of an orange oil was obtained from the obtained organic layer. 400 mL of dichloromethane (manufactured by Tokyo Chemical Industry Co., Ltd.), 400 mL methanol (manufactured by Tokyo Chemical Industry Co., Ltd.), and 8.0 mL of concentrated hydrochloric acid (manufactured by Tokyo Chemical Industry Co., Ltd.) were added thereto, the mixture was stirred at room temperature, and methanol was added to the reaction solution to produce a precipitate and obtain 37 g of a white solid. This was dissolved in 2.1 L of tetrahydrofuran (manufactured by Tokyo Chemical Industry Co., Ltd.) and concentrated under reduced pressure, and then heptane was added to the residue and the precipitated crystal was filtered. Further, the obtained product was dried under reduced pressure to obtain 35 g of an intermediate 10-3C as a target product.

### (C5 step)

A c5 step of the synthesis method (synthesis route) described above will be described.

450 mL of dehydrated tetrahydrofuran (manufactured by Tokyo Chemical Industry Co., Ltd.), 1.6 g of 4-dimethylamino pyridine (manufactured by Tokyo Chemical Industry Co., Ltd.), 11 mL of acrylic acid, and 30 g of the intermediate 10-2C were mixed in an inert atmosphere, 1.8 mL of N,N'-diisopropylcarbodiimide (manufactured by Fujifilm Wako Pure Chemical Industries, Ltd.) was added dropwise while being cooled with ice water. The ice water was removed, the mixture was stirred at room temperature, and then, Celite filtration was performed, and the filtrate was concentrated under reduced pressure to obtain 65 g of a pale yellow oil. The obtained oil, 380 mL of dehydrated tetrahydrofuran (manufactured by Fujifilm Wako Pure Chemical Industries, Ltd.), 2.1 g of 4-dimethylamino pyridine (manufactured by Tokyo Chemical Industry Co., Ltd.), and 13 mL of acrylic acid (manufactured by Tokyo Chemical Industry Co., Ltd.) were mixed, and 35 mL of N,N'-diisopropylcarbodiimide was added dropwise while being cooled with ice water. The ice water was removed, the mixture was stirred at room temperature, and then, the mixture was concentrated under reduced pressure, and chloroform and water were added to the residue and separated. After obtaining a yellow oil from an organic layer obtained by the separation, a material solidified by performing column purification and cooling was slurried with a mixed solvent of heptane/ethyl acetate (manufactured by Tokyo Chemical Industry Co., Ltd.) and filtered. The crystal was cleaned with a heptane/ethyl acetate mixed solvent, the resulting white solid was dissolved in chloroform, and 10mL of a 0.04 mg/mL phenothiazine-chloroform solution was added thereto. After that, the obtained product was concentrated to dryness to obtain 1 g of the compound represented by the chemical formula (10-3).

NMR was used to identify the structure of the compound according to Test Example 5 (the compound represented by the chemical formula (10-3)). The NMR results are as follows.

### (NMR results)

1H NMR (CDCl₃): 4.00-4.04 (4H), 4.36-4.39 (4H), 5.68-5.70 (2H), 5.77-5.81 (2H), 6.03-6.12 (2H), 6.29-6.41 (5H), 7.53-7.55 (2H), 7.64-7.70 (4H), 7.87-7.89 (2H), 8.02-8.05 (2H), 9.22-9.26 (2H).

### <Test Example 6>

### [Preparation of compound represented by chemical formula (10-4)]

The compound represented by the following chemical formula (10-4) was synthesized, and the compound represented by the following chemical formula (10-4) was used as a compound according to Test Example 6.

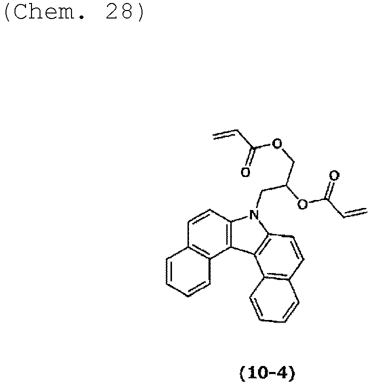

### [Method of synthesizing compound represented by chemical formula (10-4)]

The method of synthesizing (synthesis route of) the compound represented by the chemical formula (10-4) is as follows.

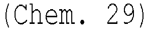

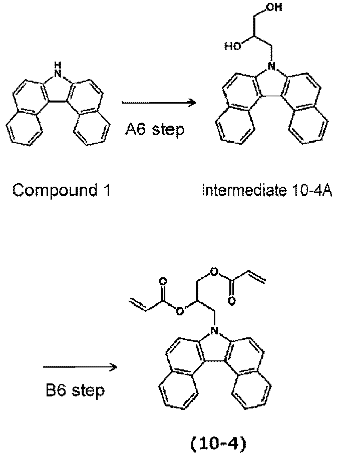

### (A6 step)

An A6 step of the synthesis method (synthesis route) described above will be described.

26 g of the compound 1 (7H-dibenzo [c, g] carbazole (manufactured by Tokyo Chemical Industry Co., Ltd.)), 400 mL of dehydrated N,N-dimethylformamide (manufactured by Fujifilm Wako Pure Chemical Industries, Ltd.), and 44.5 g of cesium carbonate (manufactured by Tokyo Chemical Industry Co., Ltd.) were mixed and stirred in an inert gas, 10 mL of glycidol (manufactured by Tokyo Chemical Industry Co., Ltd.) was added thereto, and the mixture was stirred at 70°C for several hours and was allowed to cool. The mixture was separated using water and ethyl acetate, and then column purification was performed to obtain 11 g of an intermediate 10-4A.

### (B6 step)

A B6 step of the synthesis method (synthesis route) described above will be described.

4 g of the intermediate 10-4A, 20 mL of dehydrated toluene (manufactured by Fujifilm Wako Pure Chemical Industries, Ltd.), 300 mg of 4-dimethylamino pyridine (manufactured by Fujifilm Wako Pure Chemical Industries, Ltd.), and 1.80 mL of acrylic acid (manufactured by Fujifilm Wako Pure Chemical Industries, Ltd.) were mixed in an inert gas atmosphere, 4.0 mL of N,N'-diisopropylcarbodiimide (manufactured by Fujifilm Wako Pure Chemical Industries, Ltd.) was mixed therewith in an ice-cold condition, and then, the mixture was reacted at room temperature. After that, water was added thereto, the mixture was separated with chloroform and concentrated to dryness, and column purification was performed to obtain 2.06 g of the compound represented by the chemical formula (10-4) as a target product.

NMR was used to identify the structure of the compound according to Test Example 6 (the compound represented by the chemical formula (10-4)). The NMR results are as follows.

### (NMR results)

1H NMR (CDCl₃): 4.15-4.25 (1H), 4.41-4.45 (1H), 4.83-4.86 (2H), 5.82-5.93 (2H), 6.08-6.24 (2H), 5.61-5.69 (1H), 6.34-6.43 (2H), 7.50-7.55 (2H), 7.65-7.68 (2H), 7.82-7.85 (2H), 7.91-79.5 (2H), 8.03-8.06 (2H), 9.17-9.20 (2H).

### <Test Example 7>

### [Preparation of compound represented by chemical formula (10-5)]

A compound represented by the following chemical formula (10-5) was synthesized, and the compound represented by the following chemical formula (10-5) was used as a compound according to Test Example 7.

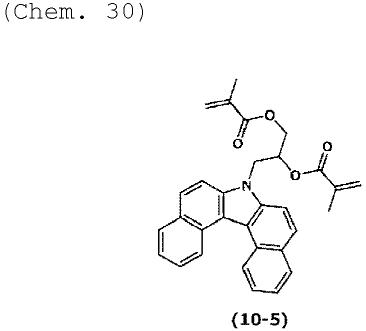

### [Method of synthesizing compound represented by chemical formula (10-5)]

The method of synthesizing (synthesis route of) the compound represented by the chemical formula (10-5) is as follows.

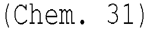

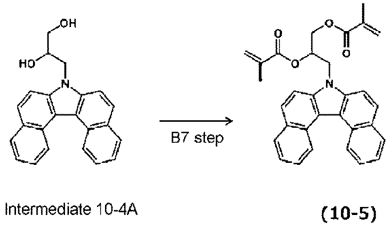

First, synthesis was advanced by using the method shown in the A6 step of the method of synthesizing (synthesis route of) of the compound represented by the chemical formula (10-4) to obtain the intermediate 10-4A.

### (B7 step)

In a B7 step of the method of synthesizing (synthesis route of) of the compound represented by the chemical formula (10-5), a compound 10-5 was obtained by using a method similar the method shown in the B6 step of the method of synthesizing (synthesis route of) the compound represented by the chemical formula (10-4) except that acrylic acid used in the B6 step of the method of synthesizing (synthesis route of) the compound represented by the chemical formula (10-4) was replaced with methacrylic acid (manufactured by Fujifilm Wako Pure Chemical Industries, Ltd.).

NMR was used to identify the structure of the compound according to Test Example 7 (the compound represented by the chemical formula (10-5)). The NMR results are as follows.

### (NMR results)

1H NMR (CDCl₃): 1.86-1.87 (3H), 1.98-1.99 (3H), 4.18-4.23 (1H), 4.42-4.40 (1H), 4.84-4.87 (2H), 5.56-5.57 (1H), 5.64-5.65 (2H), 6.07-6.18 (2H), 7.50-7.55 (2H), 7.66-7.71 (2H), 7.83-7.86 (2H), 7.92-7.95 (2H), 8.03-8.06 (2H), 9.17-9.20 (2H).

### <Test Example 8>

### [Preparation of compound represented by chemical formula (10-6)]

A compound represented by the following chemical formula (10-6) was synthesized, and the compound represented by the following chemical formula (10-6) was used as a compound according to Test Example 8.

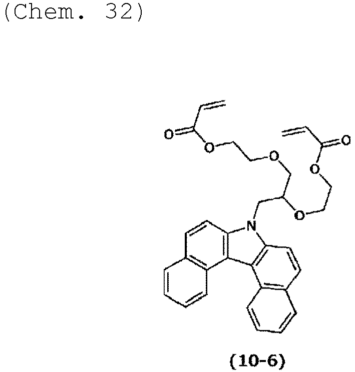

### [Method of synthesizing compound represented by chemical formula (10-6)]

The method of synthesizing (synthesis route) of the compound represented by the chemical formula (10-6) is as follows.

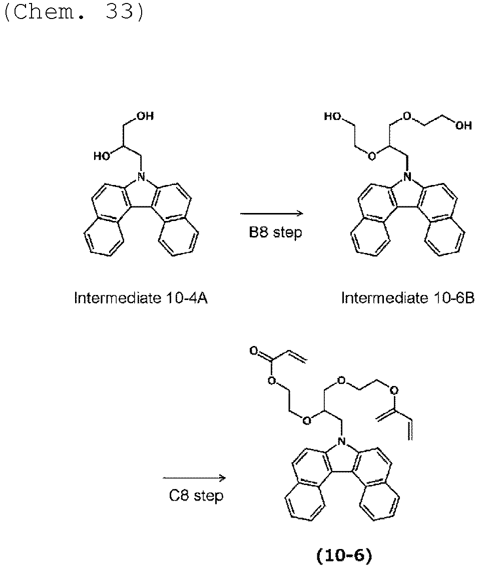

First, synthesis was advanced by using the method shown in the A6 step of the method of synthesizing (synthesis route of) the compound represented by the chemical formula (10-4) to obtain the intermediate 10-4A.

### (B8 step)

A B8 step of the synthesis method (synthesis route) described above will be described.

10 g of the intermediate 10-4A, 100 mL of 8 mol sodium hydroxide (manufactured by Fujifilm Wako Pure Chemical Industries, Ltd.), and 4 g of tetrabutylammonium bromide (manufactured by Tokyo Chemical Industry Co., Ltd.) were charged in an inert gas atmosphere and cooled in an ice bath, and then, 20 mL of 2-(2-chloroethoxy)tetrahydro-2H-pyran (manufactured by Fujifilm Wako Pure Chemical Industries, Ltd.) was mixed therewith, the cooling was stopped, and the reaction was advanced at 80°C. The mixture was separated with water and chloroform, concentrated to dryness, and dissolved in 100 mL of dichloromethane and 100 mL of methanol, 2 mL of concentrated hydrochloric acid was added thereto under ice cooling, and the mixture was stirred for 1 hour at room temperature. The obtained product was separated with water and chloroform and then concentrated to dryness under reduced pressure, and column purification was performed to obtain 6 g of an intermediate 10-6B.

### (C8 step)

A C8 step of the synthesis method (synthesis route) described above will be described.

4 g of the intermediate 10-6B, 20 mL of dehydrated toluene (manufactured by Fujifilm Wako Pure Chemical Industries, Ltd.), 300 mg of 4-dimethylamino pyridine (manufactured by Fujifilm Wako Pure Chemical Industries, Ltd.), and 1.80 mL of acrylic acid (manufactured by Fujifilm Wako Pure Chemical Industries, Ltd.) were mixed in an inert gas atmosphere, 4.0 mL of N,N'-diisopropylcarbodiimide (manufactured by Fujifilm Wako Pure Chemical Industries, Ltd.) was mixed therewith in an ice-cold condition, and the mixture was reacted at room temperature. After that, water was added thereto, the mixture was separated with chloroform and concentrated to dryness, and column purification was performed to obtain 1.7 g of the compound represented by the chemical formula (10-6) as a target product.

NMR was used to identify the structure of a compound according to Test Example 8 (the compound represented by the chemical formula (10-6)). The NMR results are as follows.

### (NMR results)

1H NMR (CDCl₃): 3.37-3.44 (1H), 3.60-3.63 (3H), 3.75-3.79 (2H), 4.00-4.02 (3H), 4.40-4.46 (2H), 4.59-4.83 (2H), 5.60-5.61 (1H), 5.79-5.87 (2H), 6.13-6.20 (2H), 6.44-6.51 (1H), 7.49-7.54 (2H), 7.63-7.70 (2H), 7.80-7.91 (4H), 8.01-8.05 (2H), 9.18-9.21 (2H).

### <Test Example 9>

### [Preparation of compound represented by chemical formula (10-7)]

A compound represented by the following chemical formula (10-7) was synthesized, and the compound represented by the following chemical formula (10-7) was used as a compound according to Test Example 9.

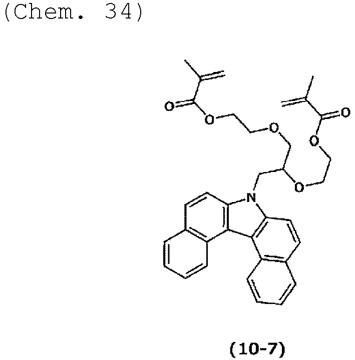

### [Method of synthesizing compound represented by chemical formula (10-7)]

The method of synthesizing (synthesis route of) the compound represented by the chemical formula (10-7) is as follows.

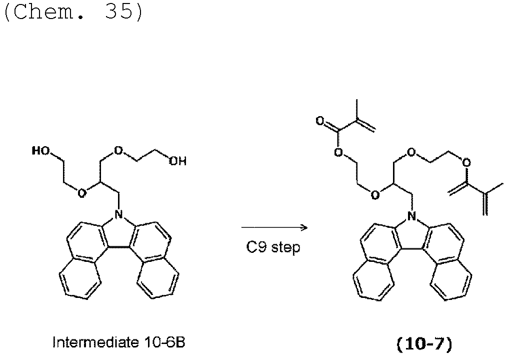

First, synthesis was advanced by using the method shown in the A6 step of the method of synthesizing (synthesis route of) the compound represented by the chemical formula (10-4) to obtain the intermediate 10-4A, and then, synthesis was advanced by using the method shown in the B8 step of the method of synthesizing (synthesis route of) the compound represented by the chemical formula (10-6) to obtain the intermediate 10-6B.

### (C9 step)

In a C9 step of the method of synthesizing (synthesis route of) the compound represented by the chemical formula (10-7), a compound 10-7 was obtained by using a method similar the method shown in the C8 step of the method of synthesizing (synthesis route of) the compound represented by the chemical formula (10-6) except that acrylic acid used in the C8 step of the method of synthesizing (synthesis route of) the compound represented by the chemical formula (10-6) was replaced with methacrylic acid (manufactured by Fujifilm Wako Pure Chemical Industries, Ltd.).

NMR was used to identify the structure of a compound according to Test Example 9 (the compound represented by the chemical formula (10-7)). The NMR results are as follows.

### (NMR results)

1H NMR (CDCl₃): 1.75-1.76 (3H), 1.96-1.97 (3H), 3.49-3.59 (1H), 3.60-3.63 (3H), 3.76-3.78 (2H), 3.93-4.04 (3H), 4.38-4.42 (2H), 4.61-4.80 (2H), 5.34-5.35 (1H), 5.57-5.58 (1H), 5.84-5.85 (1H), 6.16-6.17 (1H), 7.50-7.53 (2H), 7.65-7.68 (2H), 7.79-7.90 (4H), 8.01-8.05 (2H), 9.18-9.21 (2H).

### <Test Example 10>

### [Preparation of compound represented by chemical formula (10-8)]

A compound represented by the following chemical formula (10-8) was synthesized, and the compound represented by the following chemical formula (10-8) was used as a compound according to Test Example 10.

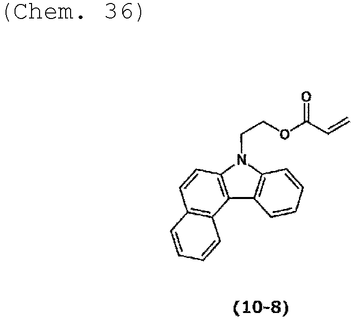

### [Method of synthesizing compound represented by chemical formula (10-8)]

The method of synthesizing (synthesis route of) the compound represented by the chemical formula (10-8) is as follows.

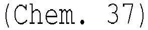

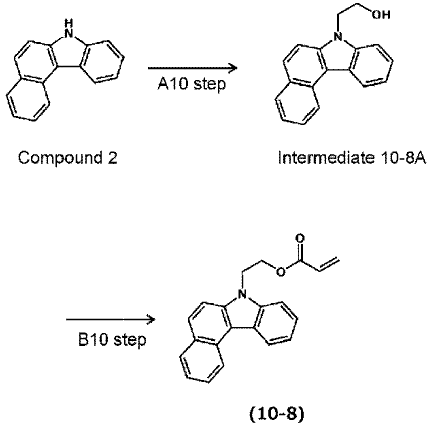

### (A10 step)

In an A10 step of the method of synthesizing (synthesis route of) the compound represented by the chemical formula (10-8), an intermediate 10-8A was obtained by using a method similar to the method shown in the A step of the method of synthesizing (synthesis route of) the compound represented by the chemical formula (6-3) except that the compound 1 (7H-dibenzo [c, g] carbazole (manufactured by Tokyo Chemical Industry Co., Ltd.)) used in the A step of the method of synthesizing (synthesis route of) the compound represented by the chemical formula (6-3) was replaced with a compound 2 (7H-benzo [c] carbazole (manufactured by Tokyo Chemical Industry Co., Ltd.)).

### (B10 step)

In a B10 step of the method of synthesizing (synthesis route of) the compound represented by the chemical formula (10-8), the compound represented by the chemical formula (10-8) was obtained by using a method similar to the method shown in the B step of the method of synthesizing (synthesis route of) the compound represented by the chemical formula (6-3) except that the intermediate 1 used in the B step of the method of synthesizing (synthesis route of) the compound represented by the chemical formula (6-3) was replaced with the intermediate 10-8A.

NMR was used to identify the structure of the compound according to Test Example 10 (the compound represented by the chemical formula (10-8)). The NMR results are as follows.

### (NMR results)

1H NMR (CDCl₃): 5.74-5.78 (1H), 5.97-6.04 (1H), 6.23-6.30 (1H), 4.74-4.78 (2H), 4.57-4.62 (2H), 7.40-7.58 (4H), 7.68-7.72 (2H), 7.90-7.93 (1H), 7.99-8.03 (1H), 8.58-8.62 (1H), 8.78-8.81 (1H).

### <Test Example 11>

### [Preparation of compound represented by chemical formula (10-9)]

A compound represented by the following chemical formula (10-9) was synthesized, and the compound represented by the following chemical formula (10-9) was used as a compound according to Test Example 11.

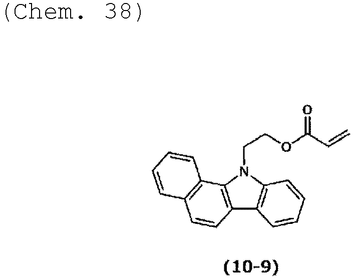

### [Method of synthesizing compound represented by chemical formula (10-9)]

The method of synthesizing (synthesis route of) the compound represented by the chemical formula (10-9) is as follows.

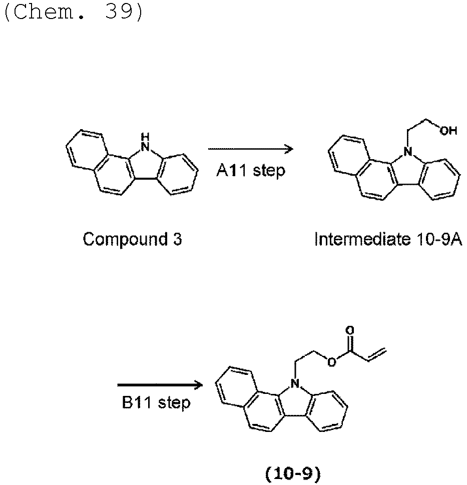

### (A11 step)

In an A11 step of the method of synthesizing (synthesis route of) the compound represented by the chemical formula (10-9), an intermediate 10-9A was obtained by using a method similar to the method shown in the A step of the method of synthesizing (synthesis route of) the compound represented by the chemical formula (6-3) except that the compound 1 (7H-dibenzo [c, g] carbazole (manufactured by Tokyo Chemical Industry Co., Ltd.)) used in the A step of the method of synthesizing (synthesis route of) the compound represented by the chemical formula (6-3) was replaced with a compound 3 (11H-benzo [a] carbazole(manufactured by Tokyo Chemical Industry Co., Ltd.)).

### (B11 step)

In a B11 step of the method of synthesizing (synthesis route of) the compound represented by the chemical formula (10-9), the compound represented by the chemical formula (10-9) was obtained by using a method similar to the method shown in the B step of the method of synthesizing (synthesis route of) the compound represented by the chemical formula (6-3) except that the intermediate 1 used in the B step of the method of synthesizing (synthesis route of) the compound represented by the chemical formula (6-3) was replaced with the intermediate 10-9A.

NMR was used to identify the structure of the compound according to Test Example 11 (the compound represented by the chemical formula (10-9)). The NMR results are as follows.

### (NMR results)

1H NMR (CDCl₃): 4.75-4.80 (2H), 5.12-5.17 (2H), 5.76-5.81 (1H), 5.98-6.26 (1H), 6.31-6.32 (1H), 7.29-7.36 (1H), 7.48-7.71 (5H), 8.04-8.20 (3H), 8.57-8.60 (1H).

### <Test Example 12>

### [Preparation of compound represented by chemical formula (10-10)]

A compound represented by the following chemical formula (10-10) was synthesized, and the compound represented by the following chemical formula (10-10) was used as a compound according to Test Example 12.

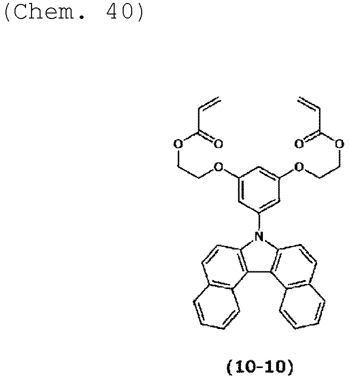

### [Method of synthesizing compound represented by chemical formula (10-10)]

The method of synthesizing (synthesis route of) the compound represented by the chemical formula (10-10) is as follows.

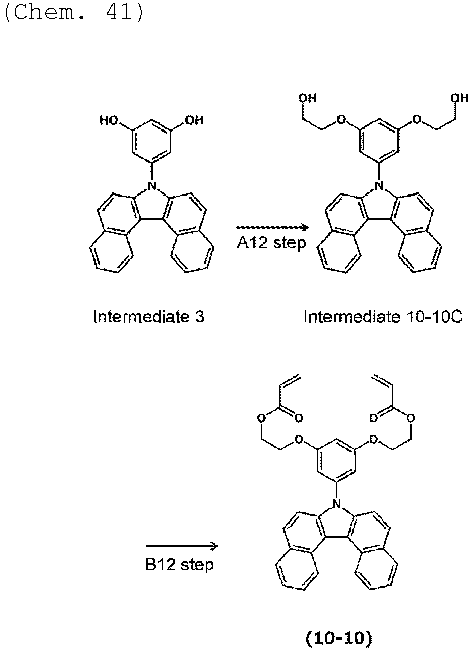

First, synthesis was advanced by using the method shown in the A1 step of the method of synthesizing (synthesis route of) the compound represented by the chemical formula (6-8) to obtain the intermediate 2, and then, synthesis was advanced by using the method shown in the B1 step of the method of synthesizing (synthesis route of) the compound represented by the chemical formula (6-8) to obtain the intermediate 3.

### (A12 step)

An A12 step of the synthesis method (synthesis route) described above will be described.

230 mL of an aqueous sodium hydroxide solution (concentration of 8.0 mol/L, manufactured by Fujifilm Wako Pure Chemical Industries, Ltd.), 8.5 g of tetrabutylammonium bromide(manufactured by Tokyo Chemical Industry Co., Ltd.), 25 g of the intermediate 3 were mixed in an inert atmosphere and cooled with ice water, and then, 46 mL of 2-(2-chloroethoxy)tetrahydro-2H-pyran (manufactured by Tokyo Chemical Industry Co., Ltd.) was added thereto. The mixture was stirred at 80°C and allowed to cool to room temperature, and water and chloroform (manufactured by Tokyo Chemical Industry Co., Ltd.) were added thereto to separate the mixture. The obtained organic layer was concentrated to dryness to obtain 66 g of a brown oil. Column purification or the like was performed on the obtained oil to obtain 17 g of an intermediate 10-10C as a target product.

### (B12 step)

A B12 step of the synthesis method (synthesis route) described above will be described.

10 mg of phenothiazine (manufactured by Tokyo Chemical Industry Co., Ltd.) was added to 50 mL of super dehydrated tetrahydrofuran (manufactured by Fujifilm Wako Pure Chemical Industries, Ltd.) to prepare a phenothiazine preparation solution. 16 g of the intermediate 10-10C and 5.0 mL of a phenothiazine preparation solution were mixed in an inert atmosphere, and 0.87 g of 4-dimethylamino pyridine (manufactured by Tokyo Chemical Industry Co., Ltd.) and 2.4 mL of acrylic acid (manufactured by Tokyo Chemical Industry Co., Ltd.) were added thereto. The mixture was cooled with ice water, and then, 15 mL of N,N'-diisopropylcarbodiimide (manufactured by Fujifilm Wako Pure Chemical Industries, Ltd.) was added thereto, and the mixture was returned to room temperature and stirred overnight. The reaction solution was filtered, and the obtained filtrate was mixed with 210 mL of super dehydrated tetrahydrofuran, 1.1 g of 4-dimethylamino pyridine, and 7.3 mL of acrylic acid. The mixture was cooled with ice water, 19 mL of N,N'-diisopropylcarbodiimide was added thereto, and the mixture was returned to room temperature and stirred overnight. The reaction solution was filtered, the filtrate was concentrated under reduced pressure, and then column purification was performed. 5.0 mL of the phenothiazine preparation solution was added to the obtained fraction and concentrated to dryness to obtain 5.9 g of the compound represented by the chemical formula (10-10).

NMR was used to identify the structure of the compound according to Test Example 12 (the compound represented by the chemical formula (10-10)). The NMR results are as follows.

### (NMR results)

1H NMR (CDCl₃): 4.24-4.26 (4H), 4.52-4.56 (4H), 5.84-5.88 (2H), 6.12-6.21 (2H), 6.42-6.49 (2H), 6.71-6.78 (3H), 7.51-7.73 (6H), 7.83-7.85 (2H), 8.02-8.05 (2H), 9.23-9.25 (2H).

### <Test Example 13>

### [Preparation of compound represented by chemical formula (10-11)]

A compound represented by the following chemical formula (10-11) was synthesized, and the compound represented by the following chemical formula (10-11) was used as a compound according to Test Example 13.

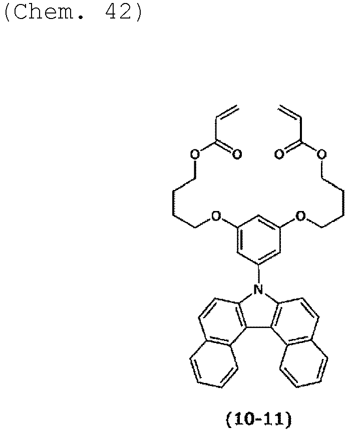

### [Method of synthesizing compound represented by chemical formula (10-11)]

The method of synthesizing (synthesis route of) the compound represented by the chemical formula (10-11) is as follows.

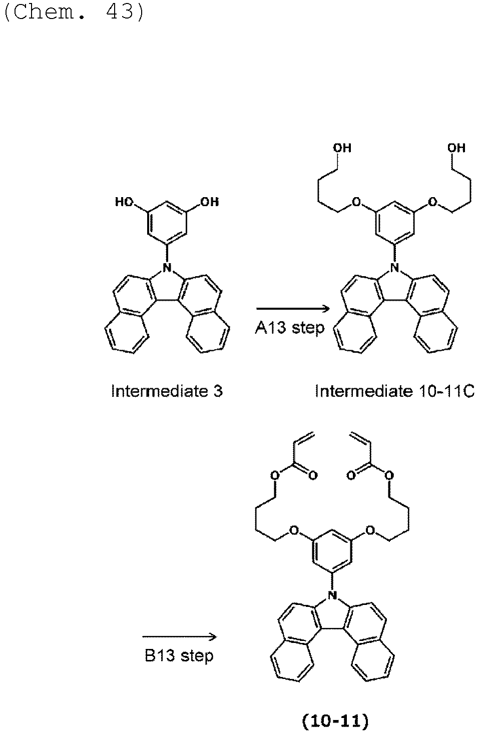

First, synthesis was advanced by using the method shown in the A1 step of the method of synthesizing (synthesis route of) the compound represented by the chemical formula (6-8) to obtain the intermediate 2, and then, synthesis was advanced by using the method shown in the B1 step of the method of synthesizing (synthesis route of) the compound represented by the chemical formula (6-8) to obtain the intermediate 3.

### (A13 step)

In an A13 step of the method of synthesizing (synthesis route of )the compound represented by the chemical formula (10-11), an intermediate 10-11C was obtained by using a method similar to the method shown in the A12 step of the method of synthesizing (synthesis route of) the compound represented by the chemical formula (10-10) except that 2-(2-chloroethoxy)tetrahydro-2H-pyran used in the A12 step of the method of synthesizing (synthesis route of) the compound represented by the chemical formula (10-10) was replaced with 2-(4-chlorobutoxy)tetrahydropyran.

### (B13 step)

In a B13 step of the method of synthesizing (synthesis route of )the compound represented by the chemical formula (10-11), the compound represented by the chemical formula (10-11) was obtained by using a method similar to the method shown in the B12 step of the method of synthesizing (synthesis route of) the compound represented by the chemical formula (10-10) except that the intermediate 10-10C used in the B12 step of the method of synthesizing (synthesis route of) the compound represented by the chemical formula (10-10) was replaced with the intermediate 10-11C.

NMR was used to identify the structure of the compound according to Test Example 13 (the compound represented by the chemical formula (10-11)). The NMR results are as follows.

### (NMR results)

1H NMR (CDCl₃): 1.90-1.92 (8H), 4.02-4.06 (4H), 4.22-4.27 (4H), 5.77-5.81 (2H), 6.06-6.14 (2H), 6.37-6.41 (2H), 6.63-6.71 (3H), 7.52-7.70 (6H), 7.83-7.85 (2H), 8.02-8.05 (2H), 9.23-9.26 (2H).

### <Test Example 14>

### [Preparation of compound represented by chemical formula (10-12)]

A compound represented by the following chemical formula (10-12) was synthesized, and the compound represented by the following chemical formula (10-12) was used as a compound according to Test Example 14.

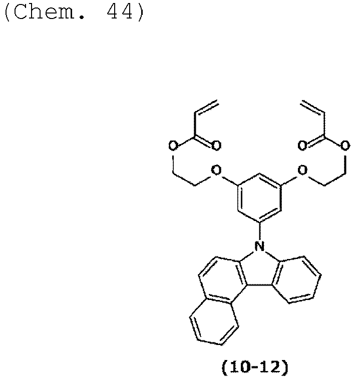

### [Method of synthesizing compound represented by chemical formula (10-12)]

The method of synthesizing (synthesis route of) the compound represented by the chemical formula (10-12) is as follows.

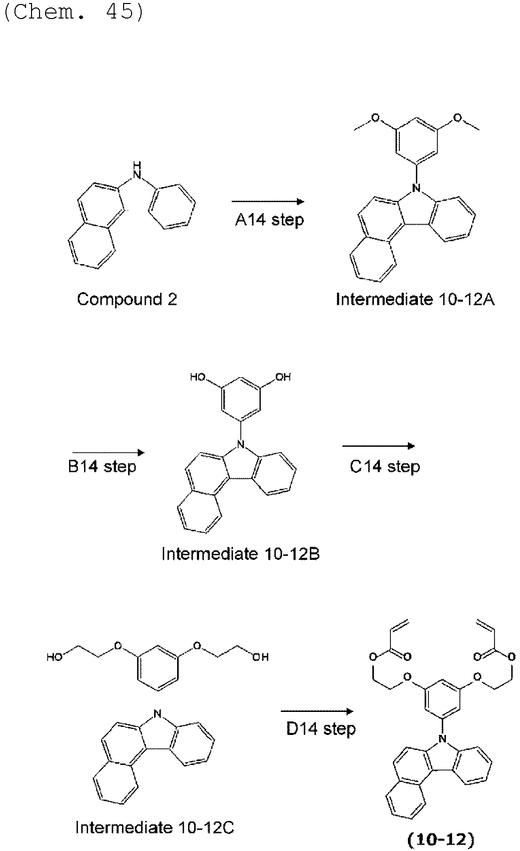

### (A14 step)

An A14 step of the synthesis method (synthesis route) described above will be described.

30 g of the compound 2 (7H-benzo [c] carbazole (manufactured by Tokyo Chemical Industry Co., Ltd.)), 60 g of 1-bromo-3,5-dimethoxybenzene (manufactured by Tokyo Chemical Industry Co., Ltd.), and 88 g of tripotassium phosphate (manufactured by Fujifilm Wako Pure Chemical Industries, Ltd.) were mixed with 738 mL of deoxidized toluene (manufactured by Fujifilm Wako Pure Chemical Industries, Ltd.) in an inert atmosphere, 33.0 mL of 1,2-cyclohexanediamine (manufactured by Tokyo Chemical Industry Co., Ltd.) and 26 g of copper iodide(I) (manufactured by Fujifilm Wako Pure Chemical Industries, Ltd.) were added thereto, the mixture was stirred at 120°C for 50 hours, 8.6 g of 1-bromo-3,5-dimethoxybenzene, 4.7 mL of 1,2-cyclohexanediamine, and 3.8 g of copper iodide (I) were added thereto, and the mixture was stirred at 120°C for 6 hours. The mixture was allowed to cool, water and chloroform (manufactured by Tokyo Chemical Industry Co., Ltd.) were added thereto, the organic layer extracted by separation was concentrated under reduced pressure, and column purification was performed on the obtained residue to obtain 38 g of an intermediate 10-12A as a target product.

### (B14 step)

A B14 step of the synthesis method (synthesis route) described above will be described.

37 g of the intermediate 10-12A was added to 420 mL of chloroform in an inert atmosphere, the mixture was cooled with ice water, and then, 420 mL of a boron tribromide-dichloromethane solution (concentration of 1.0 mol/L, manufactured by Fujifilm Wako Pure Chemical Industries, Ltd.) was added thereto, and the mixture was stirred for 3 hours. The mixture was returned to room temperature, stirred overnight, heated to 40°C, and stirred for 30 minutes. The mixture was returned to room temperature, quenched with water, and extracted with chloroform, and the organic layer was concentrated under reduced pressure. Column purification was performed on the residue, and the obtained product was concentrated under reduced pressure to obtain 23 g of an intermediate 10-12B as a target product.

### (C14 step)

A C14 step of the synthesis method (synthesis route) described above will be described.

243 mL of an aqueous sodium hydroxide solution (concentration of 8.0 mol/L, manufactured by Fujifilm Wako Pure Chemical Industries, Ltd.), 9.0 g of tetrabutylammonium bromide (manufactured by Tokyo Chemical Industry Co., Ltd.), and 23 g of the intermediate 10-12B were mixed in an inert atmosphere. The mixture was cooled with ice water, and then, 49 mL of 2-(2-chloroethoxy)tetrahydro-2H-pyran (manufactured by Tokyo Chemical Industry Co., Ltd.) was added thereto. The mixture was stirred at 80°C for 7 hours and allowed to cool to room temperature, water and chloroform (manufactured by Tokyo Chemical Industry Co., Ltd.) were added thereto, and the mixture was separated. The extracted organic layer was concentrated to dryness to obtain 87 g of a brown oil. Column purification or the like is performed on the obtained oil to obtain 20 g of an intermediate 10-12C as a target product.

### (D14 step)

A D14 step of the synthesis method (synthesis route) described above will be described.

6.6 mg of phenothiazine (manufactured by Tokyo Chemical Industry Co., Ltd.) was added to 50 mL of super dehydrated tetrahydrofuran (manufactured by Fujifilm Wako Pure Chemical Industries, Ltd.) to prepare a phenothiazine preparation solution. 16 g of the intermediate 10-12C and 9 mL of a phenothiazine preparation solution were mixed in an inert atmosphere, 1.0 g of 4-dimethylamino pyridine (Tokyo Chemical Industry Co., Ltd.) and 6.7 mL of acrylic acid(Tokyo Chemical Industry Co., Ltd.) were added thereto, the mixture was cooled with ice water, 17 mL of N,N'-diisopropylcarbodiimide(manufactured by Fujifilm Wako Pure Chemical Industries, Ltd.) was added thereto, and then, the mixture was returned to room temperature and stirred for 2 hours. The reaction solution was filtered, and the obtained filtrate was mixed with 240 mL of super dehydrated tetrahydrofuran, 1.3 g of 4-dimethylamino pyridine, and 8.4 mL of acrylic acid. The mixture was cooled with ice water, 22 mL of N,N'-diisopropylcarbodiimide was added thereto, and then, the mixture was returned to room temperature and stirred for 19 hours. Water, chloroform, and saturated saline were added to the reaction solution to extract an organic layer, the obtained organic layer was concentrated to dryness, column purification was performed on the obtained residue, and 5.0 mL of a phenothiazine preparation solution was added to the obtained fraction, and the obtained mixture was concentrated to dryness to obtain 9.6 g of the compound represented by the chemical formula (10-12).

NMR was used to identify the structure of the compound according to Test Example 14 (the compound represented by the chemical formula (10-12)). The NMR results are as follows.

### (NMR results)

1H NMR (CDCl₃): 4.26-4.27 (4H), 4.53-4.55 (4H), 5.85-5.88 (2H), 6.13-6.21 (2H), 6.44-6.48 (2H), 6.67-6.78 (3H), 7.43-7.60 (4H), 7.61-7.63 (1H), 7.70-7.72 (1H), 7.84-7.85 (1H), 7.98-8.01 (1H), 8.62-8.65 (1H), 8.83-8.85 (1H).

### <Test Example 15>

EACz (acrylic acid 2-(9H-carbazol-9-yl) ethyl (commercially available product manufactured by SIGMA-ALDRICH)) represented by the following chemical formula (11-1) was used as a compound according to Test Example 15.

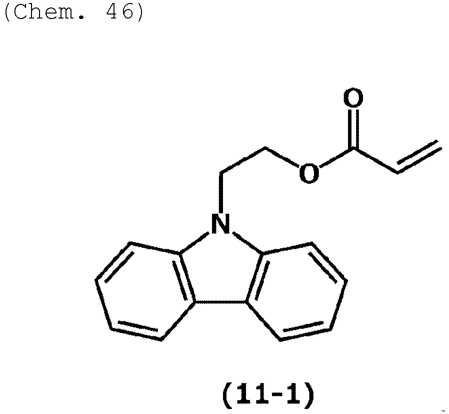

### [Method of measuring refractive index and results]

A method of measuring a refractive index and results will be described below.

An acetone solution or a chloroform solution of each of the compounds according to Test Examples 1 to 15 was prepared, and the average refractive index to light of 589 nm at room temperature of 25 ± 1 °C was measured by an Abbe refractometer (manufactured by ERMA INC., ER-1), and plotted against the volume fraction of each compound to prepare a calibration curve. Note that as the density of each compound, a value determined by a dry densitometer (AccuPyc II 1340-10CC (manufactured by Shimadzu Corporation)) was used. The calibration curve was extrapolated, and the refractive index when the volume fraction of each compound became 1 was used as the refractive index of each compound. The measurement results of the refractive index and density (g/cm³) of each of the compounds according to Test Examples 1 to 15 are shown in Table 1 below.

### [Method of evaluating transparency and results]

The visual evaluation of the color shade of each of the compounds according to Test Examples 1 to 14 showed no coloration and favorable transparency.

### [Method of measuring solubility and results]

20 mg of each of the compounds according to Test Examples 1 to 15 was weighed into a vial bottle, acetone was added to make a total amount of 10 mg, and then, the mixture was stirred with ultrasonic waves for 30 seconds. In the case where there was an undissolved product by visual recognition, a small amount of acetone was added thereto, and the mixture was stirred for another 30 seconds. The task described above was repeated, and the solubility was calculated from the total amount of solvents when all were dissolved. Note that in Test Examples 2 to 5, 7, and 10 to 11, also the solubility to chloroform was calculated in a similar way. The results of measuring the solubility (wt%) of each of the compounds according to Test Examples 1 to 15 are shown in Table 1 below.

(Table 1)

**Table 1**

| | Compound number | Density (g/cm³) | Refractive index | Solubility (wt%) | |
|---|---|---|---|---|---|
| | | | | Acetone | Chloroform |
| Test example 1 | (6 - 3) | 1. 22 | 1. 78 | 50 | - |
| Test example 2 | (6 - 8) | 1. 31 | 1. 75 | 3 | 22 |
| Test example 3 | (10 - 1) | 1. 3 | 1. 75 | 5 | 17 |
| Test example 4 | (10 - 2) | 1. 29 | 1. 73 | 20 | 33 |
| Test example 5 | (10 - 3) | 1. 29 | 1. 71 | 26 | 40 |
| Test example 6 | (10 - 4) | 1. 22 | 1. 72 | >69 | - |
| Test example 7 | (10 - 5) | 1. 26 | 1. 72 | - | 19 |
| Test example 8 | (10 - 6) | - | - | > 71 | - |
| Test example 9 | (10 - 7) | - | - | >75 | - |
| Test example 10 | (10 - 8) | 1. 3 | 1. 71 | 18 | 29 |
| Test example 11 | (10 - 9) | 1. 31 | 1. 74 | 5 | 13 |
| Test example 12 | (10 - 10) | 1. 27 | 1. 75 | >50 | - |
| Test example 13 | (10 - 11) | - | - | > 50 | - |
| Test example 14 | (10 - 12) | 1. 26 | 1. 69 | >50 | - |
| Test example 15 | (11 - 1) | 1. 21 | 1. 68 | 11 | - |

Since the compound (6-3) according to Test Example 1, the compound (6-8) according to Test Example 2, the compound (10-1) according to Test Example 3, and the compound (10-10) according to Test Example 12 each have a high refractive index, i.e., 1.75 or more, these compounds can be suitably used as high refractive index materials. Further, the refractive index of each of the compound (6-3) according to Test Example 1, the compound (6-8) according to Test Example 2, the compound (10-1) according to Test Example 3, the compound (10-2) according to Test Example 4, the compound (10-3) according to Test Example 5, the compound (10-4) according to Test Example 6, the compound (10-5) according to Test Example 7, the compound (10-6) according to Test Example 8, the compound (10-7) according to Test Example 9, the compound (10-8) according to Test Example 10, the compound (10-9) according to Test Example 11, the compound (10-10) according to Test Example 12, the compound (10-11) according to Test Example 13, and the compound (10-12) according to Test Example 14 was higher than the refractive index of the compound (11-1) according to Test Example 15 and was favorable.

Further, for example, in the case of an organic compound used as a monomer material of a hologram, it is favorable that the solubility to an organic solvent is greater than 20 wt%.

Since each of the compound (6-3) according to Test Example 1, the compound (10-4) according to Test Example 6, the compound (10-6) according to Test Example 8, the compound (10-7) according to Test Example 9, the compound (10-10) according to Test Example 12, the compound (10-11) according to Test Example 13, and the compound (10-12) according to Test Example 14 has the solubility to acetone that is a general-purpose organic solvent of greater than 20 wt%, the compound can be suitably used as a monomer material of a hologram. Further, since each of the compound (6-8) according to Test Example 2, the compound (10-2) according to Test Example 4, and the compound (10-8) according to Test Example 10 has the solubility to chloroform that is a general-purpose organic solvent of greater than 20 wt%, the compound can be suitably used as a monomer material of a hologram. Further, since the compound (10-3) according to Test Example 5 has the solubility to acetone and chloroform that are general-purpose organic solvents of greater than 20 wt%, the compound can be suitably used as a monomer material of a hologram.

### [Preparation of composition for recording hologram and hologram, and evaluation of hologram]

a composition for recording a hologram and a hologram were prepared using the compound (6-3) according to Test Example 1, the compound (10-2) according to Test Example 4, the compound (10-3) according to Test Example 5, the compound (10-8) according to Test Example 10, the compound (10-10) according to Test Example 12, the compound (10-11) according to Test Example 13, the compound (10-12) according to Test Example 14, and the compound (11-1) according to Test Example 15, and the prepared hologram was evaluated.

### <Example1> (Preparation of composition 1 for recording hologram)

In accordance with the amount shown in Table 2 below, the compound (6-3) according to Test Example 1 and bisphenoxyethanol fluorene diacrylate (manufactured by Osaka Gas Chemical Co., Ltd., "EA-0200", a refractive index: 1.62), polyvinyl acetate (manufactured by Denka Company Limited, "SN-77T"), 1,6-hexanediol diglycidyl ether (manufactured by Nagase ChemteX Corporation, "EX-212L"), Rose Bengal (manufactured by SIGMA-ALDRICH, "RB"), 4-isopropyl-4'-methyldiphenyliodonium tetrakis (pentafluorophenyl) borate (manufactured by Tokyo Chemical Industry Co., Ltd., "I0591"), and 2-mercaptobenzoxazole (manufactured by Tokyo Chemical Industry Co., Ltd., "2-MBO") as photopolymerizable monomers, a binder resin, a plasticizer, a sensitizing dye, a polymerization initiator, and a chain transfer agent were mixed in an acetone solvent at room temperature to prepare a composition 1 for recording a hologram.

### (Preparation of hologram recording medium 1)

The composition 1 for recording a hologram described above was coated on a polyvinyl alcohol film having a thickness of 2.5 µm with a bar coater so as to have a dry film thickness of 3 µm, and then a thin film surface of a photocurable resin layer was crimped onto a glass substrate having a thickness of 1.0 mm to obtain the hologram recording medium 1 obtained by stacking a glass substrate, a photocurable resin layer, and a polyvinyl alcohol film in this order.

### (Preparation of hologram 1)

Two-beam exposure was performed on the hologram recording medium 1 described above at an exposure amount of 180 mJ/cm² by using a semiconductor laser with an exposure wavelength of 532 nm, and then the entire surface was irradiated with UV (ultraviolet rays) to cure the uncured monomer, thereby fixing the refractive index distribution to the medium 1. The condition of the two-beam exposure was such that the light intensity of one beam on the recording medium was 3.0 mW/cm², the exposure was performed for 30 seconds, and the interferometric exposure was performed so that the angle between the two beams was 5.0 degrees. As a result, the refractive index distribution was formed in the hologram recording medium 1 to obtain a hologram 1.

### (Evaluation of hologram 1)

The evaluation of the refractive index modulation amount (Δn) of the prepared hologram 1 was performed in the following way.

The refractive index modulation amount (Δn) was evaluated from the maximum transmittance and full width at half maximum of the transmittance spectra obtained by entering the hologram, using the coupled-wave theory of Kogelnik (Bell System Technical Journal,48, 2909 (1969)). The transmittance spectra were obtained by measuring the transmittance at 400 to 700 nm using a spot light source manufactured by Hamamatsu Photonics as a light source and a small fiber-optic spectrometer USB-4000 manufactured by Ocean Optics, Inc. as a spectrometer.

### <Examples 2 to 5>

In Example 2, a composition 2 for recording a hologram was obtained using the same material as that in Example 1 except that the amount of the photopolymerizable monomer was changed as shown in Table 2, and by the same method as the method in Example 1 in accordance with the amount shown in Table 2.

In Example 3, a composition 3 for recording a hologram was obtained using the same material as that in Example 1 except that the amounts of the photopolymerizable monomer and the chain transfer agent were changed as shown in Table 2, Astrazon Orange G (manufactured by SIGMA-ALDRICH, "AOG") was used as a sensitizing dye, and tetrabutylammonium butyltriphenylborate (manufactured by Showa Denko, "P3B") was used as a polymerization initiator in addition to 4-isopropyl-4'-methyldiphenyliodonium tetrakis (pentafluorophenyl) borate (manufactured by Tokyo Chemical Industry Co., Ltd., "I0591"), and by the same method as the method in Example 1 in accordance with the amount shown in Table 2.

In Example 4, a composition 4 for recording a hologram was obtained using the same material as that in Example 1 except that the amount of the photopolymerizable monomer was changed as shown in Table 2, and by the same method as the method in Example 1 in accordance with the amount shown in Table 2.

In Example 5, a composition 5 for recording a hologram was obtained using the same material as that in Example 1 except that the amount of the photopolymerizable monomer was changed as shown in Table 2 and polyvinyl acetate (manufactured by Denka Company Limited, "SN-55T") was used as a binder resin, and by the same method as the method in Example 1 in accordance with the amount shown in Table 2.

### (Preparation of hologram recording mediums 2 to 5)

The compositions 2 to 5 for recording a hologram described above were used to prepare hologram recording mediums 2 to 5 by the same method as that in Example 1.

### (Preparation of holograms 2 to 5)

The hologram recording mediums 2 to 5 described above were used to prepare holograms 2 to 5 by the same method as that in Example 1 in accordance with the exposure condition shown in Table 2.

### (Evaluation of holograms 2 to 5)

The refractive index modulation amount (Δn) of each of the prepared holograms 2 to 5 was evaluated by the same method as that in Example 1.

### <Examples 6 to 13>

In Example 6, a composition 6 for recording a hologram was obtained using the same material as that in Example 1 except that the amount of the compound (6-3) that is a photopolymerizable monomer was changed as shown in table 2 and the compound (10-3) that is a photopolymerizable monomer was used in the amount shown in Table 2 without using bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 7, a composition 7 for recording a hologram was obtained using the same material as that in Example 1 except that the amount of the compound (6-3) that is a photopolymerizable monomer was changed as shown in Table 2 and the compound (10-2) that is a photopolymerizable monomer was used in the amount shown in Table 2 without using bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 8, a composition 8 for recording a hologram was obtained using the same material as that in Example 1 except that the amount of the compound (6-3) that is a photopolymerizable monomer was changed as shown in Table 2 and the compound (10-10) that is a photopolymerizable monomer was used in the amount shown in Table 2 without using bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 9, a composition 9 for recording a hologram was obtained using the same material as that in Example 1 except that the amount of the compound (6-3) that is a photopolymerizable monomer was changed as shown in Table 2 and the compound (10-12) that is a photopolymerizable monomer was used in the amount shown in Table 2 without using bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 10, a composition 10 for recording a hologram was obtained using the same material as that in Example 1 except that the compound (10-8) that is a photopolymerizable monomer was used in the amount shown in Table 2 without using the compound (6-3) that is a photopolymerizable monomer and the amount of bisphenoxyethanol fluorene diacrylate (manufactured by Osaka Gas Chemical Co., Ltd., "EA-0200", a refractive index:1.62) that is a photopolymerizable monomer was changed as shown in table 2, and by the same method as the method in Example 1 in accordance with the amount shown in Table 2.

In Example 11, a composition 11 for recording the hologram was obtained using the same material as that in Example 1 except that the compound (10-8) and the compound (10-10) that are photopolymerizable monomers were used in the amount shown in Table 2 without using the compound (6-3) that is a photopolymerizable monomer and bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 12, a composition 12 for recording a hologram was obtained using the same material as that in Example 1 except that the compound (10-8) and the compound (10-12) that are photopolymerizable monomers were used in the amount shown in Table 2 without using the compound (6-3) that is a photopolymerizable monomer and bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

In Example 13, a composition 13 for recording a hologram was obtained using the same material as that in Example 1 except that the amount of the compound (6-3) that is a photopolymerizable monomer was changed as shown in Table 2 and the compound (10-11) that is a photopolymerizable monomer was used in the amount shown in Table 2 without using bisphenoxyethanol fluorene diacrylate (manufactured by Osaka Gas Chemical Co., Ltd., "EA-0200", a refractive index: 1.62) that is a photopolymerizable monomer, and by the same method as the method in Example 1 in accordance with the amount shown in Table 2.

### (Preparation of hologram recording mediums 6 to 13)

The compositions 6 to 13 for recording a hologram described above were used to prepare hologram recording mediums 6 to 13 by the same method as that in Example 1.

### (Preparation of holograms 6 to 13)

The hologram recording mediums 6 to 13 described above were used to prepare holograms 6 to 13 by the same method as that in Example 1 in accordance with the exposure condition shown in Table 2.

### (Evaluation of holograms 6 to 13)

The refractive index modulation amount (Δn) of each of the prepared holograms 6 to 13 was evaluated by the same method as that in Example 1.

### <Comparative Example 1>

### (Preparation of composition 101 for recording hologram)

In Comparative Example 1, a composition 101 for recording a hologram was obtained using the same material as that in Example 1 except that the compound (11-1) according to Test Example 15 ((acrylic acid 2-(9H-carbazol-9-yl) ethyl (manufactured by SIGMA-ALDRICH, "EACz")) and bisphenoxyethanol fluorene diacrylate (manufactured by Osaka Gas Chemical Co., Ltd., "EA-0200", a refractive index:1.62) were used as photopolymerizable monomers in accordance with the amount shown in Table 2 below, and by the same method as the method in Example 1 in accordance with the amount shown in Table 2.

### (Preparation of hologram recording medium 101)

The composition 101 for recording a hologram described above was used to prepare a hologram recording medium 101 in the same method as that in Example 1.

### (Preparation of hologram 101)

The hologram recording medium 101 described above was used to prepare a hologram 101 by the same method as that in Example 1 in accordance with the exposure condition shown in Table 2.

### (Evaluation of hologram 101)

The refractive index modulation amount (Δn) of the prepared hologram 101 was evaluated by the same method as that in Example 1.

### <Comparative Example 2>

### (Preparation of composition 102 for recording a hologram)

In Comparative Example 2, a composition 102 for recording a hologram was obtained using the same material as that in Example 1 except that the compound (11-1) according to Test Example 15 ((acrylic acid2-(9H-carbazol-9-yl) ethyl (manufactured by SIGMA-ALDRICH, "EACz")) and bisphenoxyethanol fluorene diacrylate (manufactured by Osaka Gas Chemical Co., Ltd., "EA-0200", a refractive index: 1.62) were used as photopolymerizable monomers in accordance with the amount shown in Table 2 below, and by the same method as the method in Example 1 in accordance with the amount shown in Table 2.

### (Preparation of hologram recording medium 102)

The composition 102 for recording a hologram described above was used to prepare a hologram recording medium 102 by the same method as that in Example 1.

### (Preparation of hologram 102)

The hologram recording medium 102 descried above was used to prepare a hologram 102 by the same method as that in Example 1 in accordance with the exposure condition shown in Table 2.

### (Evaluation of hologram 102)

The refractive index modulation amount (Δn) of the prepared hologram 102 was evaluated by the same method as that in Example 1.

### <Experimental results>

The experimental results of the holograms according to Examples 1 to 13 and Comparative Examples 1 to 2 described above are shown in Table 2. Note that in Table 2, the numerical value of each component is expressed in mass%.

(Table 2)

**Table 2**

| | | | | Example 1 | Example 2 | Example 3 | Example 4 | Example 5 | Example 6 | Example 7 | Example 8 | Example 9 | Example 10 | Example 11 | Example 12 | Example 13 | Comparative Example 1 | Comparative Example 2 |
|---|---|---|---|---|---|---|---|---|---|---|---|---|---|---|---|---|---|---|
| | Photopolymerizable monomer | | | | | | | | | | | | | | | | | |
| | | Compound(6-3) | | 8. 2 | 20 | 20 | 25 | 20 | 25 | 25 | 25 | 25 | - | - | - | 25 | | |
| | | Compound(10-8) | | - | - | - | - | - | - | - | - | - | 25 | 25 | 25 | | - | |
| | | Compound(10-2) | | - | - | - | - | - | - | 20 | - | - | - | | - | - | - | |
| | | Compound(10-3) | | - | - | - | - | - | 20 | | - | - | - | - | - | - | - | |
| | | Compound(10-12) | | - | - | - | - | - | | - | - | 20 | - | - | 20 | - | - | |
| | | Compound(10-10) | | - | - | - | - | - | - | - | 20 | - | - | 20 | - | - | - | |
| | | Compound(10-11) | | - | - | - | - | - | - | - | - | - | | - | - | 20 | - | |
| | | | EACz (Compound(11-1) ) | - | - | - | - | - | - | - | - | - | - | - | | - | 20 | 8. 2 |
| | | | EA-0200 | 36. 8 | 25 | 25 | 20 | 25 | | - | - | - | 20 | - | - | - | 25 | 36. 8 |
| | Binder resin | | | | | | | | | | | | | | | | | |
| Composition | | | SN-77T | 19. 38 | 19. 38 | 19. 38 | 19. 38 | - | 19. 38 | 19. 38 | 19. 38 | 19. 38 | 19. 38 | 19. 38 | 19. 38 | 19. 38 | 19. 38 | 19. 38 |
| | | | SN-55T | - | - | - | - | 19. 38 | - | - | - | | - | | - | - | | |
| | Plasticizer | | | | | | | | | | | | | | | | | |
| | | | EX-212L | 30 | 30 | 30 | 30 | 30 | 30 | 30 | 30 | 30 | 30 | 30 | 30 | 30 | 30 | 30 |
| | Sensitizing dye | | | | | | | | | | | | | | | | | |
| | | | RB | 2. 25 | 2. 25 | - | 2. 25 | 2. 25 | 2. 25 | 2. 25 | 2. 25 | 2. 25 | 2. 25 | 2. 25 | 2. 25 | 2. 25 | 2. 25 | 2. 25 |
| | | | A0G | - | - | 2. 34 | - | - | - | - | - | - | - | | - | - | - | |
| | Polymerization initiator | | | | | | | | | | | | | | | | | |
| | | | [059] | 8. 43 | 8. 43 | 1. 4 | 8. 43 | 8. 43 | 8. 43 | 8. 43 | 8. 43 | 8. 43 | 8. 43 | 8. 43 | 8. 43 | 8. 43 | 8. 43 | 8. 43 |
| | | | P3B | - | - | 2. 28 | - | | | - | - | - | - | - | - | - | - | |
| | Chain transfer agent | | | | | | | | | | | | | | | | | |
| | | | 2-MB0 | 0. 56 | 0. 56 | 0. 14 | 0. 56 | 0. 56 | 0. 56 | 0. 56 | 0. 56 | 0. 56 | 0. 56 | 0. 56 | 0. 56 | 0. 56 | 0. 56 | 0. 56 |
| Exposure conditions | Wavelength (nm) | | | 532 | 532 | 457 | 532 | 532 | 532 | 532 | 532 | 532 | 532 | 532 | 532 | 532 | 532 | 532 |
| Evaluation | Refractive index modulation amount (Δn) | | | 0. 051 | 0. 07 | 0. 06 | 0. 075 | 0. 064 | 0. 07 | 0. 05 | 0. 08 | 0. 07 | 0. 04 | 0. 05 | 0. 05 | 0. 06 | 0. 032 | 0. 03 |

As shown in Table 2, it was found that the holograms 1 to 13 according to Examples 1 to 13 each had a high refractive index modulation amount (Δn) as compared with the hologram 101 according to Comparative Example 1 and the hologram 102 according to Comparative Example 2.

It should be noted that the embodiment according to the present technology is not limited to the embodiment described above, and various modifications can be made without departing from the essence of the present technology.

Further, the effects described herein are not limitative but are merely illustrative, and other effects may be provided.

It should be noted that the present technology may also take the following configurations.
[1] A compound represented by the following general formula (1). 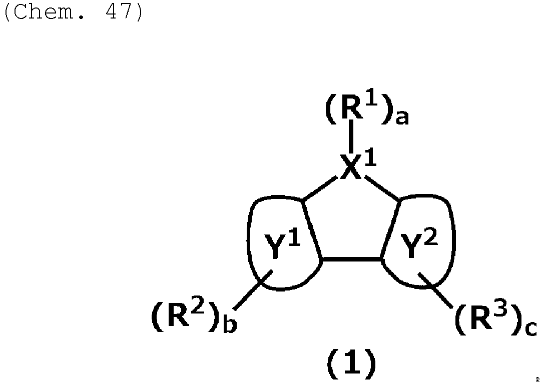
   In the general formula (1), X¹ represents an oxygen atom, a nitrogen atom, a phosphorus atom, a caron atom, or a silicon atom. Where X¹ represents an oxygen atom, where X¹ represents a nitrogen atom or a phosphorus atom, and where X¹ represents a caron atom or a silicon atom, a represents 0, a represents 1, and a represents 2, respectively.
   Y¹ and Y² each represent a benzene ring or a naphthalene ring, and both Y¹ and Y² do not represent benzene rings. Where Y¹ or Y² represents a benzene ring, b or c corresponding to Y¹ or Y² that is the benzene ring represents 4. Where Y¹ and/or Y² represent(s) a naphthalene ring(s), b and/or c corresponding to Y¹ and/or Y² that is(are) the naphthalene ring(s) represent(s) 6.
   R¹ to R³ each represent a hydrogen or a substituent group represented by *-Z¹(R⁴)_{d} (* represents a bonding site). Where R¹ to R³ respectively include a plurality of R¹, a plurality of R², and a plurality of R³, the plurality of R¹ to R³ may be the same or different from each other, but all of R¹ to R³ in the general formula (1) do not represent hydrogens.
   Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.
   R⁴ represents a hydrogen or a polymerizable substituent group. Where R⁴ includes a plurality of R⁴, the plurality of R⁴ may be the same or different from each other but all R⁴ in the general formula (1) do not represent hydrogens.
[2] The compound according to [1], in which
   X¹ in the general formula (1) represents a nitrogen atom.
[3] The compound according to [1] or [2], in which
   the compound is a compound represented by the following general formula (1-1). 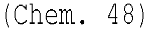 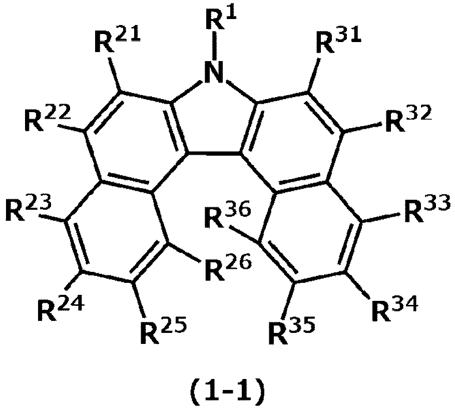
   In the general formula (1-1), R¹, R²¹ to R²⁶, and R³¹ to R³⁶ each represent a hydrogen or a substituent group represented by *-Z¹(R⁴)_{d} (* represents a bonding site). R¹, R²¹ to R²⁶, and R³¹ to R³⁶ may be the same or different from each other. However, all of R¹, R²¹ to R²⁶, and R³¹ to R³⁶ in the general formula (1-1) do not represent hydrogens.
   Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.
   R⁴ represents a hydrogen or a polymerizable substituent group. Where R⁴ includes a plurality of R⁴, the plurality of R⁴ may be the same or different from each other but all R⁴ in the general formula (1-1) do not represent hydrogens.
   However, the compound in which R¹ represents *-CH=CH₂ and all of R²¹ to R²⁶ and R³¹ to R³⁶ represent hydrogens is not included.
[4] An organic material containing the compound according to any one of [1] to [3].
[5] The organic material according to [4], in which
   the organic material is an organic thin film, an organic lens, or a hologram.
[6] The organic material according to [4], in which
   the organic material is a composition for an organic thin film, a composition for an organic lens, or a photosensitive composition for recording a hologram.
[7] A polymer obtained by polymerizing the compound according to any one of [1] to [3].
[8] An organic material containing the polymer according to [7].
[9] The organic material according to [8], in which
   the organic material is an organic thin film, an organic lens, or a hologram.
[10] The organic material according to [8], in which
   the organic material is a composition for an organic thin film, a composition for an organic lens, or a photosensitive composition for recording a hologram.
[11] An optical apparatus containing the organic material according to [4].
[12] An optical part containing the organic material according to [4].
[13] An image display apparatus containing the organic material according to [4].
[14] An optical apparatus containing the organic material according to [8].
[15] An optical part containing the organic material according to [8].
[16] An image display apparatus containing the organic material according to [8].
[17] A composition, including:
   the compound according to any one of [1] to [3]; and
   a radical polymerizable monomer.
[18] The composition according to [17], in which
   the radical polymerizable monomer is one or more selected from the group consisting of a carbazole monomer, a dinaphthothiophene monomer, a fluorene monomer, and a dibenzofuran monomer.

## Claims

1. A compound represented by the following general formula (1). 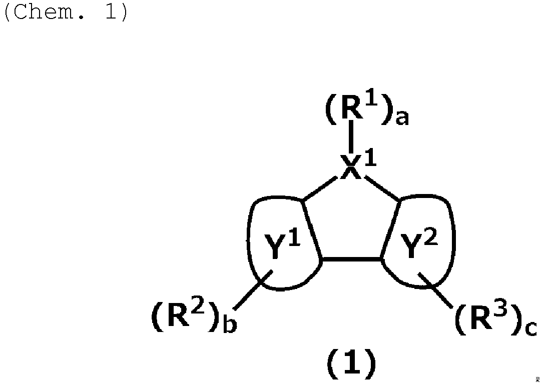
In the general formula (1), X¹ represents an oxygen atom, a nitrogen atom, a phosphorus atom, a caron atom, or a silicon atom. Where X¹ represents an oxygen atom, where X¹ represents a nitrogen atom or a phosphorus atom, and where X¹ represents a caron atom or a silicon atom, a represents 0, a represents 1, and a represents 2, respectively.
Y¹ and Y² each represent a benzene ring or a naphthalene ring, and both Y¹ and Y² do not represent benzene rings. Where Y¹ or Y² represents a benzene ring, b or c corresponding to Y¹ or Y² that is the benzene ring represents 4. Where Y¹ and/or Y² represent(s) a naphthalene ring(s), b and/or c corresponding to Y¹ and/or Y² that is(are) the naphthalene ring(s) represent(s) 6.
R¹ to R³ each represent a hydrogen or a substituent group represented by *-Z¹(R⁴)_{d} (* represents a bonding site). Where R¹ to R³ respectively include a plurality of R¹, a plurality of R², and a plurality of R³, the plurality of R¹ to R³ may be the same or different from each other, but all of R¹ to R³ in the general formula (1) do not represent hydrogens.
Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.
R⁴ represents a hydrogen or a polymerizable substituent group. Where R⁴ includes a plurality of R⁴, the plurality of R⁴ may be the same or different from each other but all R⁴ in the general formula (1) do not represent hydrogens.

2. The compound according to claim 1, wherein
X¹ in the general formula (1) represents a nitrogen atom.

3. The compound according to claim 1, wherein
the compound is a compound represented by the following general formula (1-1). 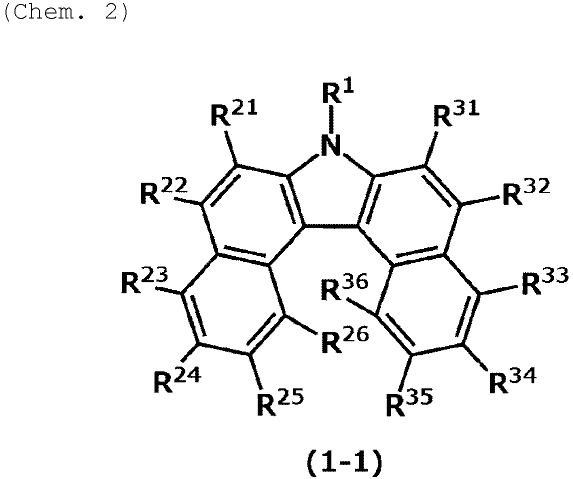
In the general formula (1-1), R¹, R²¹ to R²⁶, and R³¹ to R³⁶ each represent a hydrogen or a substituent group represented by *-Z¹(R⁴)_{d} (* represents a bonding site). R¹, R²¹ to R²⁶, and R³¹ to R³⁶ may be the same or different from each other. However, all of R¹, R²¹ to R²⁶, and R³¹ to R³⁶ in the general formula (1-1) do not represent hydrogens.
Z¹ represents a single bond, a saturated hydrocarbon group having a valence of 2 or higher, or an unsaturated hydrocarbon group having a valence of 2 or higher, and the saturated hydrocarbon group or the unsaturated hydrocarbon group may have an ether bond and/or a thioether bond. Where Z¹ represents a single bond and where Z¹ represents a saturated hydrocarbon group or an unsaturated hydrocarbon group, d represents 1 and d represents an integer of 1 or more, respectively.
R⁴ represents a hydrogen or a polymerizable substituent group. Where R⁴ includes a plurality of R⁴, the plurality of R⁴ may be the same or different from each other but all R⁴ in the general formula (1-1) do not represent hydrogens.
However, the compound in which R¹ represents *-CH=CH₂ and all of R²¹ to R²⁶ and R³¹ to R³⁶ represent hydrogens is not included.

4. An organic material containing the compound according to claim 1.

5. The organic material according to claim 4, wherein
the organic material is an organic thin film, an organic lens, or a hologram.

6. The organic material according to claim 4, wherein
the organic material is a composition for an organic thin film, a composition for an organic lens, or a photosensitive composition for recording a hologram.

7. A polymer obtained by polymerizing the compound according to claim 1.

8. An organic material containing the polymer according to claim 7.

9. The organic material according to claim 8, wherein
the organic material is an organic thin film, an organic lens, or a hologram.

10. The organic material according to claim 8, wherein
the organic material is a composition for an organic thin film, a composition for an organic lens, or a photosensitive composition for recording a hologram.

11. An optical apparatus containing the organic material according to claim 4.

12. An optical part containing the organic material according to claim 4.

13. An image display apparatus containing the organic material according to claim 4.

14. An optical apparatus containing the organic material according to claim 8.

15. An optical part containing the organic material according to claim 8.

16. An image display apparatus containing the organic material according to claim 8.

17. A composition, comprising:
the compound according to claim 1; and
a radical polymerizable monomer.

18. The composition according to claim 17, wherein
the radical polymerizable monomer is one or more selected from the group consisting of a carbazole monomer, a dinaphthothiophene monomer, a fluorene monomer, and a dibenzofuran monomer.
